# EUROPEAN PATENT APPLICATION

(11) **EP 4 455 675 A1**
(43) Date of publication of application: **30.10.2024**
(21) Application number: 22911800.5
(22) Date of filing: 19.12.2022
(51) Int. Cl.: G01N 35/00, G06N 20/00, B01L 7/00

(54) **METHOD FOR PREDICTING PERFORMANCE OF DETECTION DEVICE**

(30) Priority: 23.12.2021 KR 20210186618
(71) Applicant: Seegene, Inc., Seoul 05548 (KR)
(72) Inventor: KIM, Kwang Min, Namyangju-si, Gyeonggi-do 12251 (KR); PARK, Bong Gyun, Seoul 06069 (KR)
(74) Representative: Balder IP Law, S.L.
(86) International application number: PCT/KR2022/020688
(87) International publication number: WO 2023/121167

(57) **Abstract**

According to an embodiment of the present disclosure, method for inferring a noise level generated in a detection device includes: obtaining optic data with information about light among raw data used for a optic calibration of the detection device, wherein the detection device is used for detecting the presence or absence of a target analyte in a sample based on a signal generated dependent on the presence or absence of the target analyte; and inferring the noise level generated in the detection device by using a pre-trained machine learning model with the optic data as an input data.

## Description

### [Technical Field]

The present disclosure relates to technology related to an amplification reaction, and more particularly, to a technique for predicting the performance of a detection device.

### [Background Art]

PCR means a polymerase chain reaction and is a technology that rapidly amplifies DNA. A detection device is an equipment for performing an amplification reaction such as the PCR. At a shipping stage of the detection device, a performance test is conducted to determine whether the detection device is suitable for performing the amplification reaction such as the PCR.

A general performance test for the detection device is largely divided into a calibration step and a noise test step. Here, the calibration step is a step of calibrating the detection device with a standard detection device, and takes approximately 60 minutes. The noise test step performed after the calibration step is a major test that can confirm the performance of the detection device, but since the noise test step takes a long time, for example, 150 to 600 minutes for one device, it is necessary to research a technique which can reduce the time consumed in the noise test step.

### [Prior Art Document]

### [Patent Document]

Japanese Patent Registration No. 6897655

### [Disclosure]

### [Technical Problem]

The present disclosure has been made in an effort to provide a method for predicting a noise test result of a detection device.

Technical objects of the present disclosure are not restricted to the technical object mentioned above. Other unmentioned technical objects will be apparently appreciated by those skilled in the art by referencing the following description.

### [Technical Solution]

In order to achieve the object, according to an embodiment of the present disclosure, a method for inferring a noise level generated in a detection device which may include: obtaining optic data with information about light among raw data used for an optic calibration of the detection device, wherein the detection device is used for detecting the presence or absence of a target analyte in a sample based on a signal generated dependent on the presence or absence of the target analyte; and inferring the noise level generated in the detection device by using a pre-trained machine learning model with the optic data as an input data.

Alternatively, the optic data may include light measurement values obtained by irradiating light with a pre-determined wavelength over pre-determined cycles and light features obtained by mathematically processing the light measurement values.

Alternatively, the irradiation of light may be done to a reaction container comprising a liquid medium and a dye.

Alternatively, the optic calibration may include adjusting the intensity of the irradiated light based on a comparison result between the optic data and a predetermined reference value.

Alternatively, the optic calibration may include adjusting the light quantity measurement sensitivity of the detection device based on a comparison result between the optic data and a predetermined reference value.

Alternatively, the method further include extracting light measurement values and light features obtained by mathematically processing the light measurement values from the optic data and inputting the light measurement values and the light features to the machine learning model for inferring the noise level.

Alternatively, the light features may include at least one selected from the group consisting of a first light feature comprising a variance or standard deviation for the optic data, a second light feature comprising an average of the optic data, a third light feature comprising a trend calculated by time-series decomposition of the optic data, a fourth light feature comprising seasonality calculated by time-series decomposition of the optic data, a fifth light feature comprising a remainder obtained by subtracting the third and fourth light feature from the optic data; a sixth light feature comprising a variance of a residual calculated by applying a linear regression line to the optic data, and a combination thereof.

Alternatively, the extraction may produce a plurality of light features of different types, the machine learning model may include at least three classifiers corresponding to the light measurement values and the plurality of light features; wherein one classifier of the at least three classifiers corresponds to one light measurement values and one or at least two light features, and each of the at least three classifiers, once inputted with its corresponding light measurement values and light features, and output either PASS or FAIL as a provisional noise level of the detection device, and the machine learning model may infer the noise level of the detection device by ensembling outputs of the at least three classifiers.

Alternatively, the extraction may produce, a plurality of light features of different types, the inference of the noise level may further include extracting at least one inference feature from the light measurement value and the plurality of light features based on a predetermined p-value, and the machine learning model may be inputted with at least one inference feature to infer the noise level.

Alternatively, the machine learning model may include a model inferring the noise level by using SVM (Support vector Machine) or Partial Least Squares.

Alternatively, the machine learning model may be a supervised-learned model with a plurality of learning data, each of the plurality of learning data may include optic data obtained from selected detection devices as learning input data and include PASS or FAIL data indicating the noise level of the selected detection devices as learning answer data.

Alternatively, the noise level of the selected detection devices may be obtained with regard to negative control (NC) reaction and/or positive control (PC) reaction run on the selected detection devices.

Alternatively, the optic data may include a light measurement value and a plurality of light features of different types, the machine learning model may include a neural network model comprising an input layer, at least one hidden layer and an output layer, and the input layer may include input nodes corresponding to light measurement values and at least one of the one or more light features, and the output layer may include output nodes corresponding to PASS and FAIL.

In order to achieve the object, according to an embodiment of the present disclosure, a computer readable medium storing a computer program, in which the computer program includes commands for allowing, when the computer program is executed by one or more processors, the one or more processor to perform a method for inferring a noise level for a detection device that detects the presence or absence of a target analyte in a sample based on a signal generated dependent on the presence or absence of the target analyte, and the method may include: obtaining optic data used for a optic calibration of the detection device; and inferring the noise level generated in the detection device by using a pre-trained machine learning model with the optic data as an input data.

In order to achieve the object, according to an embodiment of the present disclosure, a computing device for performing a method for inferring a noise level generated in a detection device may include an input unit obtaining optic data used for an optic calibration of the detection device, wherein the detection device is used for detecting the presence or absence of a target analyte in a sample based on a signal generated dependent on the presence or absence of the target analyte; and a noise level inference unit inferring the noise level generated in the detection device by using a pre-trained machine learning model with the optic data as an input data.

Technical solving means which can be obtained in the present disclosure are not limited to the aforementioned solving means and other unmentioned solving means will be clearly understood by those skilled in the art from the following description.

### [Advantageous Effects]

According to an embodiment of the present disclosure, a method for testing the performance of a detection device in a shorter time than before.

Effects which can be acquired in the present disclosure are not limited to the aforementioned effects and other unmentioned effects will be clearly understood by those skilled in the art from the following description.

### [Description of Drawings]

Various aspects are now described with reference to the drawings and like reference numerals are generally used to designate like elements. In the following embodiments, for purposes of explanation, numerous specific details are set forth to provide a comprehensive understanding of one or more aspects. However, it will be apparent that the aspect(s) can be executed without the specific detailed matters. In other examples, known structures and apparatuses are illustrated in a block diagram form in order to facilitate description of the one or more aspects.
FIG. 1 is an exemplary diagram illustrating an optical calibration and testing process and a time required for each step for shipping a detection device.
FIG. 2 is a block diagram of a computing device for inferring a noise level of the detection device according to an embodiment of the present disclosure.
FIG. 3 is a configuration diagram of a processor 120 for inferring the noise level of the detection device according to an embodiment of the present disclosure.
FIG. 4 is a configuration diagram of a noise level inference unit according to an embodiment of the present disclosure.
FIG. 5 is an exemplary diagram exemplarily illustrating raw data according to some embodiments of the present disclosure.
FIG. 6 illustrates graphs decomposing an exemplary time series graph into trend, seasonality, and remainder.
FIG. 7 illustrates in a bar graph values of example light features for each of the two components according to an embodiment of the present disclosure.
FIG. 8 is an exemplary diagram exemplarily illustrating a method for generating output data in a machine learning model according to an embodiment of the present disclosure.
FIG. 9 illustrates a flowchart of a method for inferring a noise level of a detection device according to an embodiment of the present disclosure.
FIG. 10 is a simple and normal schematic view of an exemplary computing environment in which the embodiments of the present disclosure may be implemented.

### [Mode for Invention]

Various exemplary embodiments and/or aspects are now disclosed with reference to the drawings. In the description below, the plurality of particular detailed matters are disclosed for helping general understanding of one or more aspects for the purpose of description. However, the point that the aspect(s) is executable even without the particular detailed matters may also be recognized by those skilled in the art.

"Component", "module", "system", and the like which are terms used in the specification refer to a computer-related entity, hardware, firmware, software, and a combination of the software and the hardware, or execution of the software. For example, the component may be a processing process executed on a processor, the processor, an object, an execution thread, a program, and/or a computer, but is not limited thereto. For example, both an application executed in a computing device and the computing device may be the components. One or more components may reside within the processor and/or a thread of execution. One component may be localized in one computer. One component may be distributed between two or more computers. Further, the components may be executed by various computer-readable media having various data structures, which are stored therein. The components may perform communication through local and/or remote processing according to a signal (for example, data transmitted from another system through a network such as the Internet through data and/or a signal from one component that interacts with other components in a local system and a distribution system) having one or more data packets, for example.

The computing device of the present disclosure is used to encompass any type of server and/or user terminal.

Further, a term "or" intends to mean comprehensive "or" not exclusive "or". That is, unless otherwise specified or when it is unclear in context, "X uses A or B" intends to mean one of the natural comprehensive substitutions. That is, when X uses A, X uses B, or X uses both A and B, "X uses A or B" may be applied to any one among the cases. Further, a term "and/or" used in the present specification shall be understood to designate and include all of the possible combinations of one or more items among the listed relevant items.

Further, a term "comprise", "include" and/or "comprising", "including" shall be understood as meaning that a corresponding characteristic and/or a constituent element exists, but it shall be understood that the existence or an addition of one or more other characteristics, constituent elements, and/or a group thereof is not excluded. Further, unless otherwise specified or when it is unclear in context that a single form is indicated, the singular shall be construed to generally mean "one or more" in the present specification and the claims.

The term "at least one of A or B" should be interpreted to mean "a case including only A", "a case including only B", and "a case in which A and B are combined".

Those skilled in the art need to recognize that various illustrative logical blocks, configurations, modules, circuits, means, logic, and algorithm steps described in connection with the exemplary embodiments disclosed herein may be additionally implemented as electronic hardware, computer software, or combinations of both sides. To clearly illustrate the interchangeability of hardware and software, various illustrative components, blocks, configurations, means, logic, modules, circuits, and steps have been described above generally in terms of their functionalities. Whether the functionalities are implemented as the hardware or software depends on a specific application and design restrictions given to an entire system. Skilled artisans may implement the described functionalities in various ways for each specific application. However, such implementation decisions should not be interpreted as causing a departure from the scope of the present disclosure.

The description of the presented exemplary embodiments is provided so as for those skilled in the art to use or carry out the present disclosure. Various modifications of the exemplary embodiments may be apparent to those skilled in the art, and general principles defined herein may be applied to other exemplary embodiments without departing from the scope of the present disclosure. Accordingly, the present disclosure is not limited to the exemplary embodiments suggested herein, and shall be interpreted within the broadest meaning range consistent to the principles and new characteristics presented herein.

The term "target analysis substance" includes a variety of substances (e.g., biological and non-biological substances), which may refer to the same entity as the term "target analyte."

The target analytes may include specifically biological substances, more specifically at least one of nucleic acid molecules (e.g., DNA and RNA), proteins, peptides, carbohydrates, lipids, amino acids, biological compounds, hormones, antibodies, antigens, metabolites, and cells.

The target analysis substance or the target analyte, especially a target nucleic acid molecule may be amplified by various methods: polymerase chain reaction (PCR), ligase chain reaction (LCR) (US Patent No. Nos. 4,683,195 and 4,683,202; PCRProtocols: A Guide to Methods and Applications (Innis et al., eds, 1990)), strand displacement amplification (SDA) (Walker, et al. Nucleic Acids Res. 20(7):1691-6 (1992); Walker PCR Methods Appl 3(1): 1-6 (1993)), transcription-mediated amplification (Phyffer, et al., J. Clin. Microbiol. 34:834-841 (1996); Vuorinen, et al., J. Clin. Microbiol. 33:1856-1859 (1995)), nucleic acid sequence-based amplification (NASBA) (Compton, Nature 350(6313):91-2 (1991)), rollingcircle amplification (RCA) (Lisby, Mol. Biotechnol. 12(1):75-99 (1999); Hatchet al., Genet. Anal. 15(2):35-40 (1999)), and Q-Beta Replicase (Lizardi et al., BiolTechnology 6:1197(1988)), loop-mediated isothermal amplication (LAMP, Y. Mori, H. Kanda and T. Notomi, J. Infect. Chemother., 2013, 19, 404-411), recombinase polymerase amplication(RPA, J. Li, J. Macdonald and F. von Stetten, Analyst, 2018, 144, 31-67). As used herein, the term "detection device" is intended to encompass a vessel for an amplification reaction as well as equipment for the amplification reaction including a temperature controller and/or a detector. Additionally, the amplification reaction performed in the detection device in this specification means that an amplification reaction composition is amplified using an amplification reaction vessel and an amplification reaction equipment. An example of the detection device may be a nucleic acid detection device, and an example of an amplification reaction may be PCR.

In the present disclosure, a component of a nucleic acid detection device is a component that may constitute the nucleic acid detection device, may be included in the nucleic acid detection device, or may be mounted on the nucleic acid detection device, and may be, for example, a part or component of the detection device. For example, in this specification, the components of the amplification reaction device should be understood to include an amplification reaction device, an amplification reaction vessel, a reaction container, and/or a filter module, etc.

The amplification reaction device includes various types of devices that may control a temperature for an amplification reaction. Examples thereof may include CFX (Bio-Rad), iCycler (Bio-Rad), LightCycler (Roche), StepOne (ABI), 7500 (ABI), ViiA7 (ABI), QuantStudio (ABI), AriaMx (Agilent), Eco (Illumina), etc., but are not limited thereto.

The amplification reaction vessel may include a vessel including a tube, strip, plate, etc.

The above-described components are only examples, and the PCR component of the present disclosure should not be construed as being limited due to the above-described examples.

In the present disclosure, the amplification reaction vessel is a system in which the reaction is performed and may be a well plate. A reaction vessel includes one or more reaction containers. The reaction container refers to a unit that may accommodate reactants (e.g., reaction solution or reaction mixture). Test tubes, PCR tubes, strip tubes, and multi-well PCR plates are all implementation examples of reaction vessels containing one or two or more reaction containers. An example of a reaction container is a well, and the well described below may be used interchangeably with the reaction container.

The nucleic acid detection device according to the present disclosure is implemented to perform a nucleic acid amplification reaction and a nucleic acid detection task on a specimen or a sample. According to the embodiment, the nucleic acid detection device may be implemented to perform the nucleic acid detection task without performing the nucleic acid amplification reaction, or the detection device may be implemented to perform the nucleic acid amplification reaction and not perform the nucleic acid detection task, but hereinafter, the description will be made on the premise that the nucleic acid detection device is implemented to also perform the nucleic acid amplification reaction.

The nucleic acid detection device may include an optical module and a thermal module.

The optical module includes a light source module and a detection module. The light source module supplies an appropriate optical stimulation to the sample, and the detection module detects an optical signal generated from the sample in response thereto. The optical signal may be luminescence, phosphorescence, chemiluminescence, fluorescence, polarized fluorescence, or other colored signal. The optical signal may be an optical signal which gives an optical stimulation, and is generated in reaction thereto. The light source module includes a light source configured to irradiate light to the sample and a filter that filters the light irradiated from the light source.

The light source may be a light source that emits light having the same wavelength characteristics. For example, when light sources emit light in the same wavelength range, it also means that the amounts of light emitted for each wavelength range are the same as each other. The amounts of light being the same means not only that the amounts of light are completely the same, but also that the amounts of light are substantially the same. The amounts of light being substantially the same means, for example, that the same type of emitted light from an optical label is generated at the same level of light amount when light generated from both light sources is irradiated to the same optical label through the same filter. Specifically, the fact that a plurality of light sources has substantially the same wavelength characteristics means that a deviation of light amounts or wavelength ranges of the plurality of light sources is within 20%, 15%, or 10%.

A filtration filters the light emitted from the light source. The filtration means selectively passing light in a specific wavelength range among the light emitted from the light source, or not selectively passing the light in the specific wavelength range. The selectively passing the light means passing 50%, 60%, 70%, 80%, or 90% or more of a light amount in a target wavelength range. The selectively not passing means not passing, but preventing 50%, 60%, 70%, 80%, or 90% or more of the light amount of light in the target wavelength range.

The filtration of the present disclosure selectively passes the light in the specific wavelength range among the light emitted from the light source to be irradiated to the sample. As a result, only a specific optical label among the optical labels included in the sample generates the optical signal.

The optical label in this specification may be used interchangeably with dyes. For example, the dyes may be an optical label selected from the group consisting of Cy2^{™} , YO-PRO^{™} -1, YOYO^{™} -1, Calcein, FITC, FluorX^{™} , Alexa^{™} , Rhodamine 110, Oregon Green^{™} 500, Oregon Green^{™} 488, RiboGreen^{™} , Rhodamine Green^{™} , Rhodamine 123, Magnesium Green^{™} , Calcium Green^{™} , TO-PRO^{™} -1, TOTO1, JOE, BODIPY530/550, Dil, BODIPY TMR, BODIPY558/568, BODIPY564/570, Cy3^{™} , Alexa^{™} 546, TRITC, Magnesium Orange^{™} , Phycoerythrin R&B, Rhodamine Phalloidin, Calcium Orange^{™} , Pyronin Y, Rhodamine B, TAMRA, Rhodamine Red^{™} , Cy3.5^{™} , ROX, Calcium Crimson^{™} , Alexa^{™} 594, Texas Red, Nile Red, YO-PRO^{™} -3, YOYO^{™} -3, R-phycocyanin, C-Phycocyanin, TO-PRO^{™} -3, TOT03, DiD DilC(5), Cy5^{™} , Thiadicarbocyanine, Cy5.5, HEX, TET, Biosearch Blue, CAL Fluor Gold 540, CAL Fluor Orange 560, CAL Fluor Red 590, CAL Fluor Red 610, CAL Fluor Red 635, FAM, Fluorescein, Fluorescein-C3, Pulsar 650, Quasar 570, Quasar 670, and Quasar 705. The optical label includes a fluorescent label, a luminescent label, a chemiluminescent label, an electrochemical label, and a metallic label.

In particular, the dye in the present disclosure may be a fluorescent optical label selected from the group consisting of FAM, HEX, CAL Fluor Red 610, Quasar 670, and Quasar 705.

The detection module detects the signal. The detection module detects fluorescence, which is an optical signal generated from samples.

The detection module detects the optical signal by generating an electrical signal according to the intensity of the optical signal.

The detection module includes a detector configured to detect emission light emitted from the sample and a filtration filtering the emission light emitted from the sample.

The detector is configured to detect the emission light emitted from the optical label included in the sample. The detector may detect the amount of light for each wavelength by distinguishing the wavelength of light, or may detect the total amount of light regardless of the wavelength. Specifically, the detector may use, for example, a photodiode, a photodiode array, a photomultiplier tube (PMT), a CCD image sensor, a CMOS image sensor, or an avalanche photodiode (APD).

The detector is configured to detect the emission light emitted from the optical label included in the sample.

Specifically, the detector may be formed toward the sample so that the emission light generated from the sample may directly reach the detector, or toward a reflector or an optical fiber so that the emission light may reach the detector through the reflector or the optical fiber.

The filtration of the detection module is a filtration that selectively passes the emission light emitted from the optical label included in the sample. When light in another wavelength range other than the emission light emitted from the optical label included in the sample is detected by the detector, the optical signal may not be accurately detected. The detection filtration of the present disclosure allows a target to be accurately detected by selectively passing the emission light emitted from the optical label.

The thermal module is implemented to perform a nucleic acid amplification reaction. For this purpose, the thermal module includes a sample holder in which the sample is accommodated. The sample holder may include a plurality of holes, and reaction vessels may be positioned in holes. The reaction vessels may include samples, and fluorescence is emitted from the samples. The sample holder may be a conductive material. When the sample holder contacts the reaction vessels, heat may be transferred from the sample holder to the reaction vessels. For example, the sample holder may be made of metal such as aluminum, gold, silver, nickel, or copper. Alternatively, a separate component other than the sample holder may directly supply energy to the reaction vessel to control temperatures of samples in the reaction vessel. In this case, the sample holder may be configured to accommodate the reaction vessels, but not transfer heat to the reaction vessels.

One example of the sample holder is a thermal block. The thermal block may include a plurality of holes, and reaction vessels may be positioned in the holes.

Another example of the sample holder is a heating plate. The heating plate is in a form of a thin metal in contact with a plate accommodating the sample. The heating plate may be operated by a scheme in which a current is made to flow on the thin metal to heat the plate.

Yet another example of the sample holder is an accommodation unit that may accommodate one or more chips or cartridges. An example of the cartridge is a fluid cartridge that includes a flow channel.

The thermal module may additionally include a heat-generating element, a heat-dissipation plate, and a heat-dissipation fan for heating or cooling the sample holder.

Specifically, the thermal module is used for a PCR-based nucleic acid amplification reaction. More specifically, the thermal module may perform a denaturing step, an annealing step, and an extension (or amplification) step to amplify deoxyribonucleic acid (DNA) having a specific nucleotide sequence. Among them, the denaturing step is a step in which double-stranded DNA is separated into single-stranded DNA by heating a solution containing a sample and a reagent containing double-stranded DNA, which is a template nucleic acid, to a specific temperature, for example, about 95°C. The annealing step is a step in which an oligonucleotide primer having a nucleotide sequence complementary to the nucleotide sequence of the nucleic acid to be amplified is provided, and the oligonucleotide primer and the separated single-stranded DNA are cooled to a specific melting temperature (Tm), for example, 60°C, and the primer is bound to a specific nucleotide sequence of the single-stranded DNA to form a partial DNA-primer complex. The extension step is a step in which the double-stranded DNA is formed based on the primer of the partial DNA-primer complex by DNA polymerase by maintaining the solution at a specific temperature, for example, 72°C, after the annealing step.

In an embodiment, the thermal module may exponentially amplify DNA having the specific nucleotide sequence by repeating the three steps described above, for example, 10 to 50 times.

In another embodiment, the thermal module may perform the annealing step and the extension step simultaneously. In this case, the thermal module may complete one cycle by performing two steps consisting of the denaturing step and the annealing/extension step.

Specifically, the thermal module is used for an isothermal amplification reaction. More specifically, the thermal module may perform a step of maintaining a selected temperature in order to amplify a specific nucleic acid sequence. For example, the thermal module may maintain a temperature selected from 30°C to 70°C and allow the isothermal amplification reaction to be performed.

The nucleic acid detection device or detection device includes various conventionally known devices as long as the temperature may be controlled for the amplification reaction. Examples thereof may include CFX (Bio-Rad), iCycler (Bio-Rad), LightCycler (Roche), StepOne (ABI), 7500 (ABI), ViiA7 (ABI), QuantStudio (ABI), AriaMx (Agilent), Eco (Illumina), etc., but are not limited thereto.

In the present disclosure, a standard nucleic acid detection device may be designated in advance by the user and may be a reference device for calibrating other nucleic acid detection devices. The calibration process described later may include tests of calibrating another nucleic acid detection device using the standard nucleic acid detection device. Specifically, test result values output from the standard nucleic acid detection device may be used to calibrate other nucleic acid detection devices. A more detailed description of the calibration method of other nucleic acid detection device using the standard nucleic acid detection device will be described in more detail later.

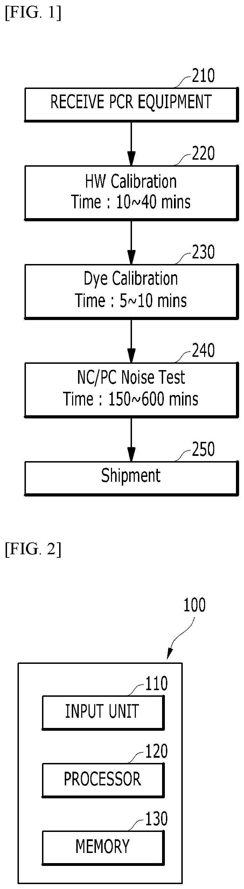

An existing method for testing the performance of the nucleic acid detection device according to some embodiments of the present disclosure will be described with reference to FIG. 1 as follows.

FIG. 1 is an example diagram illustrating the calibration and testing process and a time required for each step for shipping a nucleic acid detection device.

Specifically, a nucleic acid detection device (PCR device) is received (210), and a test to calibrate the optics of the nucleic acid detection device may include one or more optic calibration steps. For example, the test to calibrate the optics of the nucleic acid detection device may proceed to a Hardware (HW) Calibration (220) step and a Dye Calibration (230) step as illustrated in FIG. 1. Afterwards, a noise test 240 is performed on the optically calibrated nucleic acid detection device through a test for optical calibration, and the nucleic acid detection device that is determined to pass the noise test 240 is finally shipped (250). A nucleic acid detection device that is determined to fail the noise test 240 may conduct the noise test 240 again, or may be determined not to be shipped.

The noise test involves a test to confirm whether the nucleic acid detection device may be used for detection of the target analysis substance. According to an implementation example, the noise test is performed using at least one of a Negative Control composition (NC) or a Positive Control composition (PC).

Step 210 of FIG. 1 is described as an example in which the PCR device is received, but the nucleic acid detection device in the present disclosure should not be interpreted as being limited to the PCR device.

The optic calibration process of the present disclosure described later may be represented as any type of test for calibrating the optics of the nucleic acid detection device. For example, optical calibration may include any form of test performed prior to testing noise of the nucleic acid detection device. As another example, the optic calibration may include a test performed in the HW Calibration (220) step illustrated in FIG. 1. Additionally, the level of noise of the present disclosure described later may represent any form of test for the noise of the nucleic acid detection device. For example, noise level inference may be performed in the Noise Test (240) step illustrated in FIG. 1.

The optic calibration of the present disclosure may be characterized by adjusting the intensity of light irradiated from the nucleic acid detection device to the sample. Specifically, the optic calibration of the present disclosure may include a test for measuring an RFU value by irradiating light of a predetermined wavelength for a predetermined cycle to at least one component of the nucleic acid detection device or the standard nucleic acid detection device. Here, the nucleic acid detection device may be a detection device that is different from the standard nucleic acid detection device.

At least one of the plurality of wells corresponding to the component of the nucleic acid detection device may be a well containing a dye, and at least another well may be a well without a dye. The optic calibration may include a test of including such wells in the nucleic acid detection device for calibration and measuring the RFU value from the wells by irradiating light of a predetermined wavelength. The optic calibration of the present disclosure may be performed once or more for one nucleic acid detection device, and may also be performed once or more for the standard nucleic acid detection device.

The optic calibration may adjust the intensity of irradiated light based on a comparison result between the optic data and a predetermined reference value.

Additionally, in the optic calibration, at least one component of the nucleic acid detection device and the standard nucleic acid detection device may be composed of the same component. Specifically, the optic calibration may be performed on wells that are components of the standard nucleic acid detection device, and then the optic calibration may be performed on the nucleic acid detection device using the previous wells of the standard nucleic acid detection device.

For example, among 10 wells, dye may be added only to the first and second wells, and the remaining 8 wells may be wells to which no dye is added. Raw data representing RFU values for each of the wells may be obtained by performing the optic calibration on the 10 wells by irradiating light of a predetermined wavelength for a predetermined cycle through the standard nucleic acid detection device. Further, other raw data representing RFU values for each of the wells may be obtained by performing the optic calibration by irradiating light of a predetermined wavelength for a predetermined cycle through the nucleic acid detection device for optic calibration on the 10 wells used in the standard nucleic acid detection device. The optics of the detection device may be calibrated by comparing the raw data of the standard nucleic acid detection device obtained in this way with other raw data of the nucleic acid detection device.

The above-described numbers of wells are only examples, and the component of the present disclosure should not be construed as being limited due to the above-described examples. In addition, the order of performing the optic calibration for each of the above-described nucleic acid detection device and the standard nucleic acid detection device is only an example, and the order of performing the optic calibration of the present disclosure should not be interpreted as being limited due to the above-described examples.

The optic calibration may be characterized in that the measurement sensitivity of the amount of light of the detection device is adjusted based on a comparison result between the optic data and a predetermined reference value.

In the present disclosure, an light measurement value and an light feature obtained by mathematically processing the light measurement value are extracted from the optic data, and the light measurement value and the light feature are input into the machine learning model to infer the noise level.

In a different scheme from the examples described above, the optic calibration may first be performed on components of the nucleic acid detection device, and then the optic calibration may be performed by adding the components of the previous nucleic acid detection device to the standard nucleic acid detection device.

Additionally, the optic calibration of the present disclosure may include a test that compares the RFU value measured in the nucleic acid detection device with the RFU value measured in the standard nucleic acid detection device that is different from the nucleic acid detection device. Specifically, in the present disclosure, the optic calibration of the nucleic acid detection device may mean calibrating the nucleic acid detection device with the standard nucleic acid detection device. Specifically, in the present disclosure, the optic calibration may be performed on the nucleic acid detection device through the above-described optic calibration. Accordingly, the nucleic acid detection device is calibrated based on the raw data according to the optic calibration of the standard nucleic acid detection device, so that an optic calibration result may be obtained which is the same as other raw data of the optical calibration of the standard nucleic acid detection device or is higher than or similar to a predetermined similar reference. As such, the optic calibration of the present disclosure may also additionally include a test process of comparing the measured RFU values of the standard nucleic acid detection device with the RFU values of the nucleic acid detection device.

In the present disclosure, raw data is data that may be obtained from the test, and may be data representing a test result for each component used in the test of the present disclosure. For example, the raw data of the present disclosure may be one of optic calibration measurement data, and may specifically be data representing the RFU value for each of the wells, which are components on which the optic calibration is performed. The raw data may be result data of the test performed on each of the wells. Additionally, the raw data may also include RFU values measured during a predetermined cycle for each of the wells.

Further, the raw data of the present disclosure may include order information of wells that are components, and information indicating whether the wells that are the components contain dye. Further, the raw data of the present disclosure may include detection channel information to be detected by selectively detecting light in a specific wavelength range. Further, the raw data of the present disclosure may include a signal value. As used herein, the term "signal value" refers to a numerical value according to a predetermined scale of the level of the signal (e.g., signal intensity) actually measured in a cycle of a signal-generating reaction, especially an amplification reaction, or a modified value thereof. The modified value may include a mathematically processed signal value of the actually measured signal value. An example of a mathematically processed signal value of an actually measured signal value (i.e. a signal value of raw data) includes a value obtained by adding, multiplying, subtracting, or dividing the measured signal value by a selected constant, a logarithmic value, or a value of change.

According to an implementation example of the present disclosure, the signal value refers to an absolute or relative quantification value of a magnitude of a signal initially detected at the corresponding cycle number in the detector. Although the signal value may include a change value considering the relationship with other cycles, when calculating a primary change value in the method of the present disclosure, it is preferable to use a signal value excluding the change value. A signal value used in a first step of the method of the present disclosure is, compared to a primary change value and a secondary change value, also called "0th order signal value", "raw signal value", or "original signal value". The unit of the signal value may vary depending on the type of signal generation reaction used. For example, when a signal value is obtained at each cycle through a real-time PCR amplification reaction, the signal value may be a value expressed in Relative Fluorescence Unit (RFU).

Further, the raw data of the present disclosure may include an light feature acquired by mathematically processing the signal value.

In the present disclosure, a cycle refers to, in a plurality of measurements accompanied by a change in a certain condition, a change unit of the condition. The change in certain condition means, for example, an increase or decrease in temperature, reaction time, reaction number, concentration, pH, and copy number of the measurement target (for example, nucleic acid). Thus, a cycle can be a time or process cycle, a unit operation cycle, and a reproductive cycle. The term cycle may refer to one unit of repetition when a reaction of a certain process is repeated or a reaction is repeated at a certain time interval.

For example, in the case of the polymerase chain reaction (PCR), one cycle refers to a reaction that includes denaturation of nucleic acid, hybridization or annealing of nucleic acid and primer, and extension of the primer. In this case, the change in certain condition is an increase in the number of repetitions of the reaction, and the repetition unit of the reaction including the series of steps is set as one cycle. That is, in the cycle of the present disclosure, for example, the test may be performed on a first well for a predetermined number of times, 3 times, in a first cycle, which is one cycle. As another example, the test may be repeatedly performed on the first well a predetermined number of times, 10 times, in the first cycle, which is one cycle.

The examples of the cycle, well, and a predetermined number of times should not be construed as limiting the cycle, well, and the predetermined number of times of the cycle of the present disclosure.

As used herein, the term "cycle section" refers to a specific cycle region consisting of a predetermined number of consecutive cycles in a data set. Each of the plurality of cycle sections has a start cycle and an end cycle. The plurality of cycle sections may be cycle sections of the same size made up of the same number of cycles, cycle sections of different sizes made up of different numbers of cycles, or cycle sections of the same size and cycle sections of different sizes may also be mixed. For example, the entire data set may be divided into multiple cycle sections of the same size, each consisting of 8 cycles. Alternatively, a baseline region of the data set may be divided into cycle sections composed of 10 consecutive cycles, and an exponential phase region may be divided into cycle sections composed of 6 consecutive cycles.

In the present disclosure, inferring the level of noise is a test to confirm the performance of the nucleic acid detection device, and may be a test of whether the nucleic acid detection device has appropriate performance or reaches an appropriate level to perform the nucleic acid detection reaction.

Specifically, inferring the level of noise means may be a test for at least one of whether a noise signal is output from the nucleic acid detection device itself or measuring the magnitude of the output noise signal when performing the nucleic acid detection reaction on a random test subject through the nucleic acid detection device.

More specifically, inferring the level of noise may be a noise test related to at least one of the Negative Control (NC) reaction or Positive Control (PC) reaction for the nucleic acid detection device. Here, the Negative Control reaction and the Positive Control reaction may be control groups used in experiments according to general scientific methodology. Specifically, the Negative Control reaction is a negative control group and may be a sample that is predicted to yield no test result. In other words, a sample predicted to have a test result different from a test result of an experimental group that is the subject of the experiment may be the negative control group. A reaction mixture is a reaction mixture containing oligos and enzymes.

NC does not contain the target analysis substance in the reaction mixture.

Further, the Positive Control reaction is a positive control group and may be a sample that is predicted to yield a test result. In other words, a sample predicted to have a test result which is the same as the test result of the experimental group may be the positive control group.

PC contains the target analysis substance in the reaction mixture. The performance of the nucleic acid detection device in the present disclosure may be any type of indicator indicating whether the nucleic acid detection device is suitable for performing the PCR. For example, the performance of the nucleic acid detection device of the present disclosure may also be determined based on whether the nucleic acid detection device itself does not output the noise signal or the magnitude of the noise signal. The presence or absence of a noise signal output from the nucleic acid detection device itself or the quantitative value of the noise signal may be measured through the evaluation of the noise described above.

As used herein, the term noise refers to an unwanted, non-analyte related signal that is generated regardless of the presence or absence of a target analyte. For example, noise includes electrical-noise generated by the measuring equipment itself, regardless of whether the signal generating reaction is executed, which is recorded as a signal; and noise in the baseline region in the nucleic acid amplification curve. Therefore, the term noise has a different meaning from an abnormal signal, which includes a sudden increase or decrease in signal.

As an example, the noise of the present disclosure may refer to a signal generated from the nucleic acid detection device itself and not related to PCR analysis. When the user later performs PCR analysis using a nucleic acid detection device that outputs a noise signal or a large noise signal, an incorrect nucleic acid amplification reaction analysis result may also be provided to the user. To prevent this, in the present disclosure, the noise level of the nucleic acid detection device itself may be determined before shipping the nucleic acid detection device by inferring the noise level described later.

In the present disclosure, raw data may be raw data obtained from the optic calibration. For example, the raw data may be raw data for each component obtained from optical calibration performed on one nucleic acid detection device. Additionally, in order to predict the performance of the nucleic acid detection device in the present disclosure, the raw data may be used to predict the level of noise.

In the present disclosure, optic data containing information about light may be obtained from the raw data used for the optic calibration of the nucleic acid detection device.

Here, the optic data may include an light measurement value obtained by irradiating light of a predetermined wavelength during a predetermined cycle and an light feature obtained by mathematically processing the light measurement value.

Additionally, inference data may be input to a machine learning model to infer the noise level, and here, the inference data may be generated using the raw data.

Here, inferring the noise level means inferring the level of noise generated from the (nucleic acid) detection device by comparing the level of noise with a predetermined value or level. For example, when the noise level is equal to or higher than the predetermined value or level, the corresponding detection device is a pass detection device, and when the noise level is equal to or lower than the predetermined value or level, the corresponding detection device is a fail detection device.

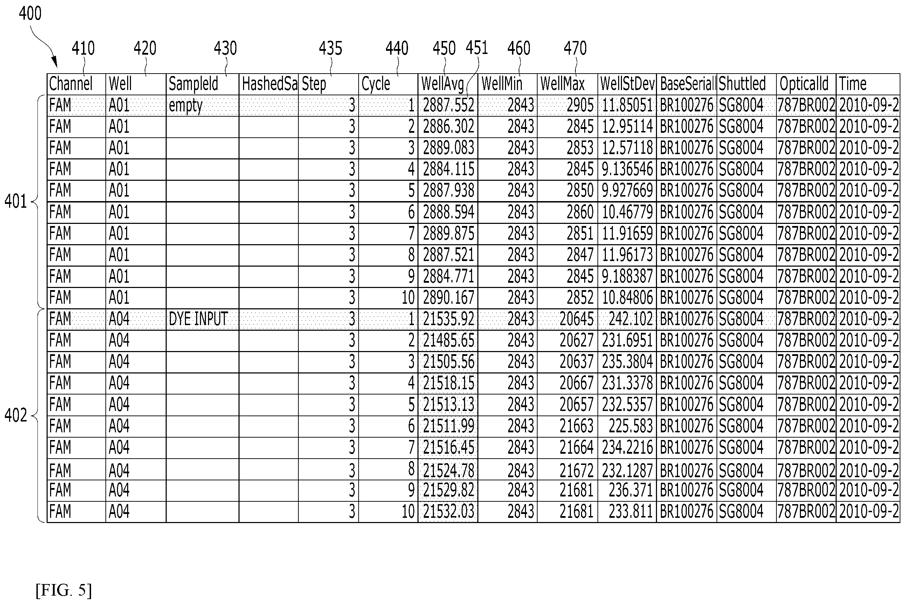

The raw data of the present disclosure is first described with reference to FIG. 5.

FIG. 5 is an exemplary diagram exemplarily illustrating raw data according to some embodiments of the present disclosure.

As illustrated in FIG. 5, the raw data 400 may include first raw data 401 and second raw data 402. The first raw data 401 and the second raw data 402 may include one or more parameters, and the raw data 400 may also include one or more parameters.

Specifically, as illustrated in FIG. 5, the parameters included in the raw data 400 may include a first parameter 410 representing a predetermined wavelength, a second parameter 420 representing a well as a component, a third parameter 430 representing whether the dye is included in the component, a fourth parameter 440 indicating a cycle, a fifth parameter 450 indicating an average of RFU values measured in one cycle, a sixth parameter 460 indicating a minimum value of the RFU values measured in one cycle, a seventh parameter 470 indicating a maximum value of the RFU value measured in one cycle, and an eighth parameter 435 indicating measuring the RFU value n times, preferably three times in one cycle. In addition, although not indicated by a separate reference number in FIG. 5, the raw data 400 of the present disclosure may further include other parameters indicating time, channel, etc.

The above-described parameters and the parameters illustrated in FIG. 5 are only examples, and the features that may be included in the raw data of the present disclosure should not be construed as being limited due to the above-described examples.

In the present disclosure, variable data may be data representing values corresponding to the above-described features. That is, the variable data may be data containing values corresponding to features. Accordingly, the raw data may include variable data corresponding to the included features, respectively.

As an example, variable data of a light feature may indicate the name of the light feature and a value corresponding to the light feature.

Specifically, with reference to FIG. 5, for example, the raw data 400 may include one or more fifth parameters 450 that represent the average of the corresponding RFU value when the RFU measurement is repeated n times, preferably 3 times, in one cycle. In addition, the raw data 400 may also include one or more first variable data 451 representing average values when the RFU measurement is repeated n times, preferably 3 times, in one cycle corresponding to the fifth parameter 450.

The above-described feature and variable data are only examples, and the feature and variable data of the present disclosure should not be construed as being limited due to the above-described examples.

In the present disclosure, the light feature may be a parameter related to noise of the measurement device among the parameters of the raw data described above. Additionally, the light feature may be a parameter or value that may be calculated from RFU values. That is, the light feature may be a parameter or value obtained by mathematically processing the light measurement value, which is the RFU value.

Specifically, the light feature may be a parameter highly related to the performance of the nucleic acid detection device, or may be a parameter highly related to noise generation from the nucleic acid detection device itself. Specifically, the light feature may be a variance of residuals calculated in RFU values based on trend, seasonality, and remainder, or a first-order simple linear regression line calculated by signal decomposition for parameters highly related to noise generation, for example, the variance of RFU values, the standard deviation of RFU values, the average of RFU values, and a time series graph of RFU values.

The above-described parameters are only examples, and the parameters highly related to noise generation of the present disclosure should not be construed as being limited due to the above-described examples.

The above-described trends, seasonality, and remainder may be data elements used to describe time series patterns in time series data analysis in statistics. Specifically, the trend, seasonality, and remainder may be expressed as the time series graph, and the trend may be expressed as a fluctuating pattern in data that increases or decreases over a long period of time. Further, the seasonality may be expressed as a pattern shown at a predetermined period of time. In addition, the remainder may be expressed as a pattern excluding the above-described trend and seasonality, and may be a random element.

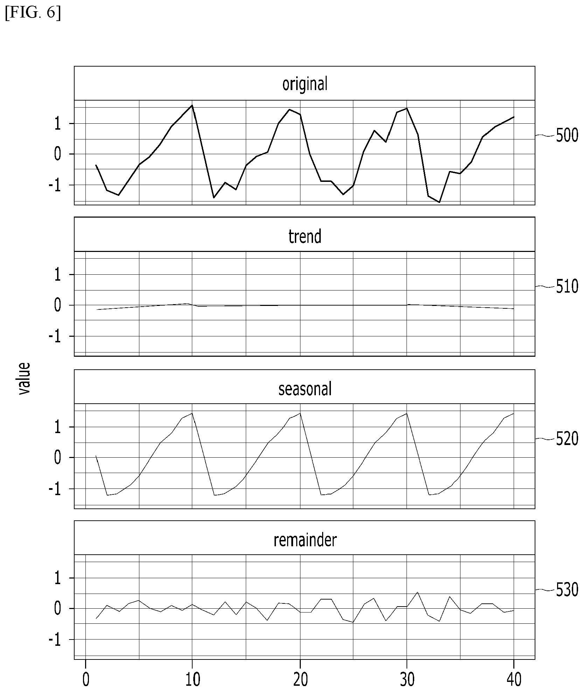

Specifically, the trend, seasonality, and remainder of the present disclosure are described with reference to FIG. 6.

FIG. 6 illustrates graphs decomposing an exemplary time series graph into trend, seasonality, and remainder.

As illustrated in FIG. 6, when the time series data such as the above-described raw data is displayed as a graph, the time series data may be displayed as a time series graph 500. Further, when decomposing the time series graph 500 into a time series, the trend of the time series graph 500 may be represented by a first graph 510, the seasonality may be represented by a second graph 520, and the remainder may be represented by a third graph 530. That is, when the first graph 510, the second graph 520, and the third graph 530 are recombined, the recombined graph may be expressed as the original time series graph 500.

The graphs in FIG. 6 only illustrate examples of the graph, trend, seasonality, and remainder of time series data, and the time series data, trend, seasonality, and remainder of the present disclosure should not be construed as being limited to FIG. 6.

Additionally, depending on the parameters described above, the light feature of the present disclosure may include a first light feature representing the variance or standard deviation of the measured RFU values for a component of the nucleic acid detection device. Specifically, the first light feature may represent the variance or standard deviation of the RFU values measured at each of the predetermined cycles for the raw data. That is, the first light feature may represent the variance or standard deviation of the RFU values measured at each of the predetermined cycles of a test conducted on one component of the nucleic acid detection device.

For example, when the calibration process is performed for a predetermined 45 cycles for one first well, the first raw data of the first well may include an average, a maximum value, and a minimum value of the RFU values measured in each of the first to 45-th cycles. Here, the average of the RFU values measured in the first cycle may be 2000, the maximum value may be 3000, and the minimum value may also be 1000.Further, the average of the RFU values measured in the second cycle may be 2500, the maximum value may be 4000, and the minimum value may also be 1000.

In the present disclosure, the average, maximum, and minimum values of the RFU values refer to the average, maximum, and minimum values of signal values measured repeatedly n times, preferably three times, within one cycle. This is because the RFU may be measured 10 times or more in each cycle, that is, within one cycle.

As such, the first raw data may also represent the average, maximum, and minimum values of the RFU values measured in each of the first to 45th cycles. Further, the first light feature for the first raw data may be the variance or standard deviation calculated for each of the above-described first to 45th cycles. Specifically, the variance or standard deviation of each cycle may be calculated based on the average, maximum, and minimum values of the RFU values measured in each of the first to 45th cycles, and here, 45 first light features may be calculated.

The above-described cycle, and the average, maximum, and minimum values of the RFU values are only examples, and the cycle, RFU values, raw data, or first light feature of the present disclosure should not be construed as being limited to the above-described examples.

In addition, the light feature of the present disclosure may also include a second light feature representing an average of RFU values for one component of the nucleic acid detection device. Specifically, the second light feature may represent an average of the RFU values for each of the predetermined cycles for the raw data.

For example, the calibration process may also be performed for each of the wells for predetermined 10 cycles. In addition, RFU values may be measured by performing a test a predetermined number of times in one cycle out of 10 predetermined cycles. Based on the RFU values of each of the first to tenth cycles, the average RFU value of each cycle may be calculated. That is, a first average of the RFU values measured in the first cycle may be calculated, and a second average of the RFU values measured in the second cycle may be calculated. The average calculated in this way is the second light feature, and the second light feature may be one of the parameters included in the raw data. In addition, in this example, since the average value calculated for 10 cycles is 10, 10 second light features may also be calculated corresponding to each of the 10 cycles.

The above-described cycle is only an example, and the cycle, raw data, or second light feature of the present disclosure should not be construed as being limited to the above-described example.

In addition, the light feature of the present disclosure may also include a third light feature representing a trend calculated by time-series decomposing the raw data, a fourth light feature representing seasonality calculated by time-series decomposing the raw dataset, and a fifth light feature representing a remainder other than the trend and seasonality calculated by time-series decomposing the raw dataset.

Specifically, raw data representing RFU values measured over predetermined cycles of the present disclosure may be time series data, and such raw data may be decomposed to calculate the trend, seasonality, and remainder. The average and variance of the trend calculated herein may be included in the third light feature, the average and variance of the seasonality may be included in the fourth light feature, and the average and variance of the remainder may be included in the fifth light feature.

Further, the light feature of the present disclosure may include a sixth light feature including the variance of the residual calculated by applying the linear regression line to the optic data.

Here, the variance of the residuals is calculated using the general calculation method used to calculate the variance of the residuals in regression analysis.

In addition, the light feature of the present disclosure may correspond to one component and one raw data, and may be a parameter already included in the raw data. For example, as described above, the average value of the RFU values for one cycle may already be included in one raw data. Here, a parameter representing the average value included in the raw data may be extracted as the first light feature.

The above-described first light feature and cycle are only examples, and the parameters or cycles that may be extracted with the light features of the present disclosure should not be construed as being limited to the above-described examples.

Specifically, with reference to FIG. 5, for example, the raw data 400 may include one or more fifth parameters 450 that represent the average of the corresponding RFU value every cycle. In addition, the raw data 400 may also include one or more first variable data 451 representing average values for each cycle to correspond to the fifth parameter 450. In this case, the fifth parameter 450 may be identified as the first light feature, and the first variable data 451 may be variable data corresponding to the first light feature.

The parameter and variable data in FIG. 5 described above are only examples, and the parameter or variable data included in the raw dataset of the present disclosure should not be construed as being limited due to the above-described examples and FIG. 5.

In the present disclosure, inference data is data input to the machine learning model to predict the level of noise, and may include data highly related to the performance of the nucleic acid detection device. Specifically, at least one parameter included in the raw data may be selected as the light feature. In addition, at least one of the light features may be selected as an inference feature based on a predetermined p-value and generated as inference data of the present disclosure. Accordingly, the inference data of the present disclosure may be data including one or more inference features selected based on a predetermined p-value.

Accordingly, one inference data may include a light measurement value selected from raw data, one or more light features, and variable data corresponding thereto, and here, the inference feature included in the inference data may be a light measurement value and a light feature additionally selected based on the predetermined p-value. The predetermined p-value of the present disclosure is described in detail later.

In the present disclosure, one inference data may correspond to one component, or one inference data may correspond to one nucleic acid detection device including a plurality of components. Inference data which is not specifically specified throughout this specification for the present disclosure refers to inference data for one nucleic acid detection device.

In the present disclosure, output data may be data representing the result of an evaluation of inferred noise. Specifically, the output data of the present disclosure is output through the machine learning model and may include a result predicting whether the nucleic acid detection device will pass the level for noise following the optic calibration. In addition, the output data may include a result of predicting whether to omit the test for noise following the optic calibration for the nucleic acid detection device.

In the present disclosure, at least one of the raw data or the inference data may be input to the machine learning model to infer the noise level. Preferably, the computing device of the present disclosure that performs the method of inferring the noise level of the nucleic acid detection device may input the raw data itself into the machine learning model. In addition, the computing device of the present disclosure may preprocess raw data to generate inference data, and input the generated inference data into the machine learning model.

In the present disclosure, a p-value is a significance probability of statistical hypothesis verification used in the statistics, and is generally used to prove the reliability of experimental data. In the field of research, the p-value of the experimental data is the statistical probability of how many values of the current experimental data will be observed under the assumption that one hypothesis is correct. Specifically, in the research field, the p-value for the experimental hypothesis established based on the experimental result data is obtained, and the experimental hypothesis is verified based on whether the p-value is higher than a predetermined criterion. The predetermined criterion for the p-value may be determined based on a confidence interval of the population, experimental data, but is usually set at 5%, i.e., 0.05. The p-value of the present disclosure, which will be described later, is also set to 0.05, which is a general standard, but the p-value of the present disclosure is not limited to 0.05.

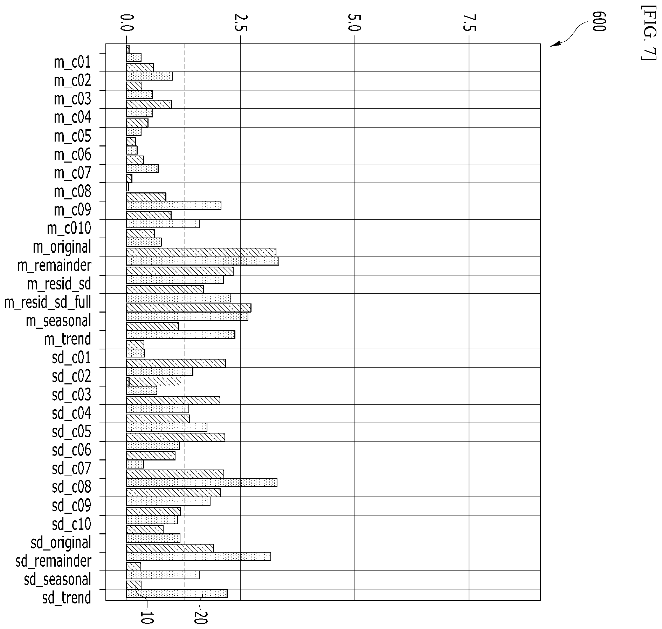

Further, the p-value of the present disclosure is an example of any type of statistics-based reference values used as a reference for selecting some features highly related to the performance of the nucleic acid detection device among the parameters of the raw data. Specific contents thereof will be described below in detail with reference to FIG. 7. The machine learning model of the present disclosure may be a model for predicting the result of noise evaluation based on the measurement result of the calibration process. Specifically, the machine learning model of the present disclosure may be a model for predicting whether the noise level passes or fails based on at least a portion of the optic data containing information about light among the raw data that is the result of the optic calibration. Here, passing the noise level is equivalent to passing a predetermined level or value, and may mean that the nucleic acid detection device has excellent performance and is in a state of being capable of shipment. Further, failing the noise level is equivalent to not passing a predetermined level or value, and may mean that the nucleic acid detection device has an inferior performance and is not in the state of being capable of shipment. Inferring the evaluation result of the nucleic acid detection device thereafter can be seen as inferring the noise level of the nucleic acid detection device.

As a result, the machine learning model of the present disclosure may be a model that outputs output data using Support Vector Machine (SVM) or Partial Least Squares.

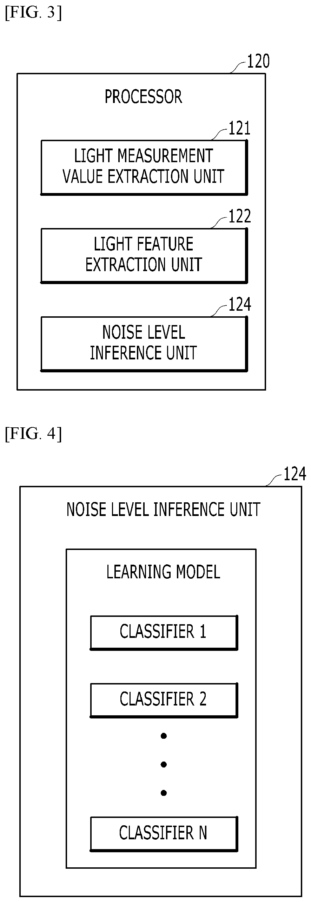

FIG. 4 is a configuration diagram of a noise level inference unit according to an embodiment of the present disclosure. Referring to FIG. 4, the noise level inference unit 124 includes a machine learning model.

The machine learning model of the present disclosure may include one or more of at least three classifiers corresponding to the light measurement value and a plurality of types of light features. Specifically, the machine learning model may include a classifier corresponding to the light measurement value, and one or more classifiers corresponding to variable data of respective light features. Further, the machine learning model of the present disclosure may include classifiers whose number corresponds to the number of predetermined combinations of inference features. Specifically, the machine learning model may include one or more classifiers corresponding to variable data of respective inference features.

Each of the at least three classifiers may be implemented to receive one light measurement value and one light feature, but may also be implemented to receive at least two light features.

Each of the at least three classifiers may output either pass or fail as a provisional noise level of the detection device when corresponding light measurement values and light features are input. In an additional embodiment of the present disclosure, the present disclosure may predict the result for the evaluation of the noise by using a disjoint clustering algorithm. For example, a computing device may generate an output that predicts the level of noise based on input inference data or raw datasets by using K-means clustering.

Regarding a learning scheme of the K-means Clustering, each group in the K-means Clustering may have one centroid, and the input learning data may be assigned to a cluster belonging to the nearest centroid. When K, the number of clusters, is specified, the K-means Clustering may be used in a learning or an iterative manner using K as a hyperparameter.

Learning or iteration of the K-means Clustering may be performed based on, for example, an expectation-maximization (EM) algorithm. For example, the computing device may repeat a process of randomly determining the centroid of the cluster, performing clustering based on the determined centroid, and calculating the average value of data included in the clustered cluster. The computing device may perform clustering on a learning dataset by continuing the above-described iterations until a situation is reached where the result does not change even with additional iterations.

Throughout this specification, the classifier, a computational model, a neural network model, a neural network, a network function, and a neural network may be used interchangeably and the above-described expressions may be included within the category of the machine learning model. The neural network may be generally constituted by an aggregate of calculation units that are mutually connected to each other, which may be called nodes. The nodes may also be called neurons. The neural network is configured to include one or more nodes. The nodes (alternatively, neurons) constituting the neural networks may be connected to each other by one or more links.

In the neural network, one or more nodes connected through the link may relatively form the relationship between an input node and an output node. Concepts of the input node and the output node are relative and a predetermined node which has the output node relationship with respect to one node may have the input node relationship in the relationship with another node and vice versa. As described above, the relationship of the input node to the output node may be generated based on the link. One or more output nodes may be connected to one input node through the link and vice versa.

In the relationship of the input node and the output node connected through one link, a value of data of the output node may be determined based on data input in the input node. Here, a link connecting the input node and the output node to each other may have a weight. The weight may be variable and the weight is variable by a user or an algorithm in order for the neural network to perform a desired function. For example, when one or more input nodes are mutually connected to one output node by the respective links, the output node may determine an output node value based on values input in the input nodes connected with the output node and the weights set in the links corresponding to the respective input nodes.

As described above, in the neural network, one or more nodes are connected to each other through one or more links to form a relationship of the input node and output node in the neural network. A characteristic of the neural network may be determined according to the number of nodes, the number of links, correlations between the nodes and the links, and values of the weights granted to the respective links in the neural network. For example, when the same number of nodes and links exist and there are two neural networks in which the weight values of the links are different from each other, it may be recognized that two neural networks are different from each other.

The neural network may be constituted by a set of one or more nodes. A subset of the nodes constituting the neural network may constitute a layer. Some of the nodes constituting the neural network may constitute one layer based on the distances from the initial input node. For example, a set of nodes of which distance from the initial input node is n may constitute n layers. The distance from the initial input node may be defined by the minimum number of links that should be passed through to reach the corresponding node from the initial input node. However, the definition of the layer is predetermined for description and the order of the layer in the neural network may be defined by a method different from the aforementioned method. For example, the layers of the nodes may be defined by the distance from a final output node.

The initial input node may mean one or more nodes in which data is directly input without passing through the links in the relationships with other nodes among the nodes in the neural network. Alternatively, in the neural network, in the relationship between the nodes based on the link, the initial input node may mean nodes that do not have other input nodes connected through the links. Similarly thereto, the final output node may mean one or more nodes that do not have the output node in the relationship with other nodes among the nodes in the neural network. Further, a hidden node may mean nodes constituting the neural network other than the initial input node and the final output node.

In the neural network according to an embodiment of the present disclosure, the number of nodes of the input layer may be the same as the number of nodes of the output layer, and the neural network may be a neural network of a type in which the number of nodes decreases and then, increases again from the input layer to the hidden layer. Further, in the neural network according to another embodiment of the present disclosure, the number of nodes in the input layer may be smaller than the number of nodes in the output layer, and the neural network may be a neural network of a type in which the number of nodes decreases from the input layer to the hidden layer. Further, in the neural network according to yet another embodiment of the present disclosure, the number of nodes of the input layer may be larger than the number of nodes of the output layer, and the neural network may be a neural network of a type in which the number of nodes increases from the input layer to the hidden layer. The neural network according to still yet another embodiment of the present disclosure may be a neural network of a type in which the neural networks are combined.

A deep neural network (DNN) may refer to a neural network that includes a plurality of hidden layers in addition to the input and output layers. When the deep neural network is used, the latent structures of data may be determined. That is, latent structures of photos, text, video, voice, and music (e.g., what objects are in the photo, what the content and feelings of the text are, what the content and feelings of the voice are) may be determined. The deep neural network may include a convolutional neural network (CNN), a recurrent neural network (RNN), an autoencoder, generative adversarial networks (GAN), a restricted Boltzmann machine (RBM), a deep belief network (DBN), a Q network, a U network, a Siam network, a Generative Adversarial Network (GAN), and the like. The description of the deep neural network described above is just an example and the present disclosure is not limited thereto.

In an embodiment of the present disclosure, the network function may include the autoencoder. The autoencoder may be a kind of artificial neural network for outputting output data similar to input data. The autoencoder may include at least one hidden layer and odd hidden layers may be disposed between the input and output layers. The number of nodes in each layer may be reduced from the number of nodes in the input layer to an intermediate layer called a bottleneck layer (encoding), and then expanded to the output layer (symmetrical to the input layer) symmetrical to a reduction in the bottleneck layer. The autoencoder may perform a non-linear dimensional reduction. The number of input and output layers may correspond to a dimension after preprocessing the input data. The autoencoder structure may have a structure in which the number of nodes in the hidden layer included in the encoder decreases as the distance from the input layer increases. When the number of nodes in the bottleneck layer (a layer having a smallest number of nodes positioned between an encoder and a decoder) is too small, a sufficient amount of information may not be delivered, and as a result, the number of nodes in the bottleneck layer may be maintained to be a specific number or more (e.g., half of the input layers or more).

The neural network and the machine learning model may be learned in at least one scheme of supervised learning, unsupervised learning, semi supervised learning, or reinforcement learning. The learning of the neural network and the machine learning model may be a process in which the neural network applies knowledge for performing a specific operation to the neural network or the machine learning model. Contents regarding learning and learning data of the neural network described below may also be contents regarding learning and learning data of the machine learning model of the present disclosure.

The neural network may be learned in a direction to minimize errors of an output. The learning of the neural network is a process of repeatedly inputting learning data into the neural network and calculating the output of the neural network for the learning data and the error of a target and back-propagating the errors of the neural network from the output layer of the neural network toward the input layer in a direction to reduce the errors to update the weight of each node of the neural network. In the case of the supervised learning, the learning data labeled with a correct answer is used for each learning data (i.e., the labeled learning data) and in the case of the unsupervised learning, the correct answer may not be labeled in each learning data. That is, for example, the learning data in the case of the supervised learning related to the data classification may be data in which category is labeled in each learning data. The labeled learning data is input to the neural network, and the error may be calculated by comparing the output (category) of the neural network with the label of the learning data. As another example, in the case of the unsupervised learning related to the data classification, the learning data as the input is compared with the output of the neural network to calculate the error. The calculated error is back-propagated in a reverse direction (i.e., a direction from the output layer toward the input layer) in the neural network and connection weights of respective nodes of each layer of the neural network may be updated according to the backpropagation. A variation amount of the updated connection weight of each node may be determined according to a learning rate. Calculation of the neural network for the input data and the back-propagation of the error may constitute a learning cycle (epoch). The learning rate may be applied differently according to the number of repetition times of the learning cycle of the neural network. For example, in an initial stage of the learning of the neural network, the neural network ensures a certain level of performance quickly by using a high learning rate, thereby increasing efficiency and uses a low learning rate in a latter stage of the learning, thereby increasing accuracy.

In learning of the neural network, the learning data may be generally a subset of actual data (i.e., data to be processed using the learned neural network), and as a result, there may be a learning cycle in which errors for the learning data decrease, but the errors for the actual data increase. Overfitting is a phenomenon in which the errors for the actual data increase due to excessive learning of the learning data. For example, a phenomenon in which the neural network that learns a cat by showing a yellow cat sees a cat other than the yellow cat and does not recognize the corresponding cat as the cat may be an example of overfitting. Overfitting may act as a cause which increases the error of the machine learning algorithm. Various optimization methods may be used in order to prevent overfitting. In order to prevent overfitting, a method such as increasing the learning data, regularization, dropout of omitting a part of the node of the network in the process of learning, utilization of a batch normalization layer, etc., may be applied.

FIG. 2 is a block diagram of a computing device for inferring a noise level of the nucleic acid detection device according to an embodiment of the present disclosure.

A configuration of the computing device 100 illustrated in FIG. 2 is only an example shown through simplification. In an embodiment of the present disclosure, the computing device 100 may include other components for performing a computing environment of the computing device 100, and only some of the disclosed components may also constitute the computing device 100.

The computing device 100 of the present disclosure may include an input unit 110, a processor 120, and a memory 130.

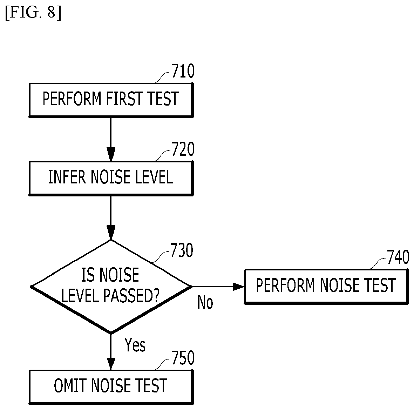

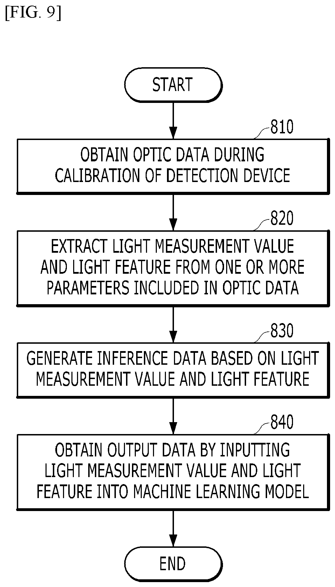

The processor 120 may be constituted by one or more cores and may include processors for data analysis, which include a central processing unit (CPU), a general purpose graphics processing unit (GPGPU), a tensor processing unit (TPU), and the like of the computing device. The processor 120 may read a computer program stored in the memory 130 to perform data processing for machine learning according to an embodiment of the present disclosure. For example, the processor 120 may perform steps described in FIGS. 8 and 9 of the present disclosure.

At least one of the CPU, GPGPU, and TPU of the processor 120 may process learning of a network function or the machine learning model. For example, both the CPU and the GPGPU may process the learning of the network function or the machine learning model, or data classification using the network function or the machine learning model. Further, in an embodiment of the present disclosure, processors of a plurality of computing devices may be used together to process the learning of the network function or the machine learning model and the data classification using the network function or the machine learning model. Further, the computer program executed in the computing device according to an embodiment of the present disclosure may be a CPU, GPGPU, or TPU executable program.

According to an embodiment of the present disclosure, the memory 130 may store any type of information generated or determined by the processor 120 or any type of information received by the input unit 110.

According to an embodiment of the present disclosure, the memory 130 may include at least one type of storage medium of a flash memory type storage medium, a hard disk type storage medium, a multimedia card micro type storage medium, a card type memory (for example, an SD or XD memory, or the like), a random access memory (RAM), a static random access memory (SRAM), a read-only memory (ROM), an electrically erasable programmable read-only memory (EEPROM), a programmable read-only memory (PROM), a magnetic memory, a magnetic disk, and an optical disk. The computing device 100 may operate in connection with a web storage performing a storing function of the memory 130 on the Internet. The description of the memory is just an example and the present disclosure is not limited thereto.

In the present disclosure, the input unit 110 may be configured regardless of communication modes such as wired and wireless modes and constituted by various communication networks including a personal area network (PAN), a wide area network (WAN), and the like. Further, the network may be known World Wide Web (WWW) and may adopt a wireless transmission technology used for short-distance communication, such as infrared data association (IrDA) or Bluetooth.

The techniques described in this specification may also be used in other networks in addition to the aforementioned networks.

FIG. 3 is a configuration diagram of a processor 120 for inferring the noise level of the nucleic acid detection device according to an embodiment of the present disclosure.

Referring to FIG. 3, the processor 120 includes a light measurement value extraction unit 121, a light feature extraction unit 122, and a noise level inference unit 124.

The light measurement value extraction unit 121 extracts the light measurement value from optic data.

The light feature extraction unit 122 extracts light features obtained by mathematically processing the light measurement values from optic data.

The noise level inference unit 124 infers the noise level generated from the nucleic acid detection device using a pre-trained machine learning model using a light measurement dataset as an input.

The processor 120 of the present disclosure may acquire raw data generated from optic calibration for calibrating the optics of the nucleic acid detection device.

The light measurement value extraction unit 121 may extract an RFU value, which is a light measurement value, from optical data containing information about light among the raw data.

The light feature extraction unit 122 may extract at least one parameter related to the noise of the nucleic acid detection device from the raw data. Specifically, the light feature extraction unit 122 may extract one or more parameters that may be used to predict the level of noise from the raw data. The parameters extracted in this way may be selected as the light features described above.

The light feature extraction unit 122 of the present disclosure may generate inference data to be used to predict the level of noise based on at least one light feature. Specifically, the light feature extraction unit 122 may obtain the extracted light feature, and generate the inference data based on the light feature. As an example, the inference data may be generated by a scheme of applying a statistical reference value to the light features. A p-value exists as an example of a statistical reference value.

The noise evaluation result extraction unit 124 of the present disclosure may obtain output data predicting the noise level by inputting inference data or raw datasets to the machine learning model.

The above-described optic calibration, noise level, raw dataset, light features, inference data, output data, and machine learning model are described in detail with reference to the following drawings.

Hereinafter, a method for predicting the performance of the nucleic acid detection device and a computing device that performs the method will be described with reference to the drawings.

The processor 120 may obtain raw data generated from optic calibration for calibrating the optics of the nucleic acid detection device. More specifically, the processor 120 may obtain a raw dataset including raw data related to RFU obtained by irradiating light of a predetermined wavelength for a predetermined cycle to at least one component of the nucleic acid detection device.

According to an embodiment of the present disclosure, the processor 120 may extract at least one parameter related to the noise of the nucleic acid detection device from the raw data through the light measurement value extraction unit 121 and the light feature extraction unit 122, and determine the extracted parameter as the light feature. As described above, the light feature of the present disclosure may include at least one of the first light feature, the second light feature, the third light feature, the fourth light feature, the fifth light feature, and the sixth light feature.

Additionally, the light feature of the present disclosure may also further include a seventh light feature representing an average of standard deviations calculated for respective predetermined cycles of RFU values with respect to the component of the nucleic acid detection device. Further, the light feature of the present disclosure may also include an eighth light feature representing a total average of RFU values for all predetermined cycles for the component of the nucleic acid detection device.

For example, when the predetermined cycle is 20 cycles, an average of standard deviations for a total of cycles may be calculated by dividing the sum of the standard deviations calculated for each of the 20 cycles in the raw data by 20. Here, the average of the standard deviations for the total of cycles may be included in the seventh light feature. The above-described cycles are only examples, and the cycle of the present disclosure should not be construed as being limited due to the above-described example.

According to another embodiment of the present disclosure, the processor 120 may also generate interference data to be used for predicting the level of noise based on at least one light feature selected before through the light feature extraction unit 122. Here, noise level prediction may be a test related to the noise of the nucleic acid detection device as described above, and the optic calibration and the noise level prediction may be different tests.

Specifically, based on a predetermined p-value, the processor 120 may select, as an inference feature, a light feature whose relevance to the performance of the nucleic acid detection device is equal to or higher than a threshold among at least one extracted parameter. Here, the processor 120 of the present disclosure may generate the inference data based on the selected inference feature and variable data corresponding thereto. Specifically, processor 120 of the present disclosure may generate the inference data based on the variable data of the light feature selected as the inference feature through the inference data generation module 160.

Here, the threshold is a reference value related to the p-value, and with reference to FIG. 7, selecting at least some of the light features as the inference features based on the p-value will be described as follows.

FIG. 7 illustrates in a bar graph values of example light features for each of the two components according to an embodiment of the present disclosure.

Specifically, an x-axis of the bar graph 600 shown in FIG. 7 represents a first light feature and a second light feature for a first component 10 and a second component 20 for each predetermined cycle, and represents third to eighth light features.

Specifically, the light features displayed on the x-axis of the graph shown in FIG. 7 are described as follows. The predetermined cycles are indicated as "c01" to "c 10", indicating a total of 10 cycles. Accordingly, "sd_c01" to "sd_c010" represent the first light feature, which is the standard deviation of the RFU values measured in each cycle, and "m_c01" to "m_c010" represent the second light feature, which is the average of the RFU values measured in each cycle. "sd_original" represents the seventh light feature, which is the average of the standard deviations of each of the cycles, and, as described above, represents the average calculated based on the standard deviations of each of 1 to 10 cycles. "m_original" represents the eighth light feature, which is the total average calculated based on the averages of each of the predetermined cycles, and the eighth light feature is as described above. "m_trend" represents the average of the third light feature indicating a trend, and "sd_trend" represents the standard deviation of the third light feature. "m_seasonal" represents the average of the fourth light feature indicating seasonality, and "sd_seasonal" represents the standard deviation of the fourth light feature. "m_remainder" represents the average of the fifth light feature representing the remainder in time series decomposition, and "sd_remainder" represents the standard deviation of the fifth light feature representing the remainder in time series decomposition. "m_resid_sd" represents the average of the regression line residuals, and "sd_resid_sd" represents the standard deviation of the regression line residuals.

The above-described light features are only examples, and the light feature of the present disclosure should not be construed as being limited due to the above-described examples.

Further, a y-axis of the graph 600 shown in FIG. 7 represents variable data corresponding to the light feature. Here, when the predetermined p-value is 0.05, the y-axis may be displayed as a dotted line illustrated in FIG. 7, and a light feature with a value higher than the y-axis value corresponding to the dotted line may be selected as an inference feature.

Specifically, "m_c01" indicated on the x-axis of the graph 600 in FIG. 7 represents the first light feature of each of the first component 10 and the second component 20 for one cycle, where the y-axis value of "m_c01" may be variable data corresponding to the first light feature and may be an average RFU value of each of the first component 10 and the second component 20 for one cycle. In addition, since the y-axis value of "m_c01" in FIG. 7 corresponds to a value lower than the dotted line, the first light feature for one cycle may not be selected as the inference feature.

In addition, "m_c03" indicated on the x-axis of the graph 600 in FIG. 7 represents the first light feature of each of the first component 10 and the second component 20 for 3 cycles. Here, the y-axis value of "m_c03" corresponds to a higher value than the dotted line, so the first light feature for 3 cycles may be selected as the inference feature, and the y-axis value of "m_c03" in FIG. 7 may be included in the inference data.

As described above, the selected light feature and variable data corresponding to the light feature may be generated as the inference data. The light feature selected here may be the inference feature of the present disclosure described above.

The contents described above with reference to FIG. 7 are merely examples, and the light features or corresponding variable data of the present disclosure should not be construed as being limited due to the above-described examples.

According to an embodiment of the present disclosure, the processor 120 may input inference data or raw data into a machine learning model and obtain output data that predicts the level of noise. Specifically, with reference to FIG. 8, a method for generating output data from the machine learning model is described as follows.

FIG. 8 is an exemplary diagram exemplarily illustrating a method for generating output data in a machine learning model according to an embodiment of the present disclosure.

Specifically, a method illustrated in FIG. 8 may be performed to predict the performance of the nucleic acid detection device and determine whether to omit the test for noise. More specifically, as described above in FIG. 1, optic calibration may be preferably performed (710) during the HW Calibration (220) process. Accordingly, the processor 120 may input optic data obtained through optic calibration to the machine learning model. For instance, performing a first test (710) includes performing the optic calibration.

Additionally, according to an embodiment of the present disclosure, the noise level may be inferred(720) based on the optic data obtained through the optic calibration.

According to an embodiment of the present disclosure, the machine learning model can predict(730) whether the nucleic acid detection device passes the noise level following optic calibration. The machine learning model or machine learning-based classifier of the present disclosure may refer to a model or classifier that provides an output for input data based on machine learning. For example, the machine learning model or machine learning-based classifier of the present disclosure may be a model that outputs output data using Support Vector Machine (SVM) or Partial Least Squares (PLS) as described above. The machine learning model or machine learning-based classifier may include a model pre-trained through a learning dataset.

The SVM of the present disclosure is a support vector machine, one of the machine learning fields, and is a supervised learning model mainly used for data classification and regression analysis. Specifically, the SVM algorithm defines one or more decision boundaries, which are classification baselines, to classify categories or classes of data, and classifies the dataset based on the defined decision boundaries.

In addition, the partial least squares (PLS) of the present disclosure is one of the dimensionality reduction methods and is a method of extracting a linear combination variable with a high covariance with a dependent variable. The partial least squares (PLS) is especially useful in regression analysis when predictor variables are closely related to each other or when the number of predictor variables is greater than the number of observations (cases).

The machine learning model of the present disclosure may output a prediction result for the evaluation result of noise based on inference data or raw data through at least one of the SVM or partial least squares algorithm.

In an additional embodiment of the present disclosure, the machine learning model may connect the SVM and the partial least squares algorithm in series in the form of using the output of the SVM as the input of the partial least squares algorithm, or using the output of the partial least squares algorithm as the input of the SVM.

In an additional embodiment of the present disclosure, the machine learning model inputs the same inference data and optic data into each of the SVM and partial least squares algorithms in parallel, and combines or ensembles the output of the SVM and the output of the partial least squares to output the prediction result.

According to an embodiment of the present disclosure, the processor 120 may input optic data containing information about light among the obtained raw data into the machine learning model. In addition, according to another embodiment of the present disclosure, the processor 120 may generate inference data based on optic data and input the generated inference data into the machine learning model.

Here, the inference data and the raw data include a plurality of variable data as described above, and the machine learning model may include a plurality of classifiers corresponding to each of the light measurement value and the light feature from the inference feature of the inference data, and the optic data having the information about the light in the raw data. That is, the machine learning model may include a plurality of classifiers corresponding to the inference features of the inference data, respectively. Alternatively, the machine learning model may include at least three classifiers corresponding to the light measurement value and light features in the raw data.

Each of at least three classifiers in the present disclosure may correspond to the SVM or the partial least squares algorithm. Specifically, one classifier may correspond to the light measurement value.

Further, one classifier may correspond to one variable data or one light feature. In this case, each of at least three classifiers may predict a result of pass or fail for the noise level based on the corresponding light measurement value and variable data of the light feature and output the predicted result as the prediction result. Further, the machine learning model of the present disclosure may generate output data that predicts the noise level by ensembling the output for each of at least three classifiers.

For example, the first inference data of the first component may include the first variable data of the first inference feature representing the average value over three cycles and the fourth variable data of the fourth inference feature representing the seasonality average. In this case, the machine learning model may include a first classifier corresponding to the first variable data and a fourth classifier corresponding to the fourth variable data. In addition, a first prediction result of predicting pass or fail of the evaluation of the noise based on the first variable data is output through the first classifier, and a second prediction result of predicting pass or fail of the evaluation of the noise based on the fourth variable data may be output through the fourth classifier.

The above-described inference data, inference feature, variable data, and classifier are only examples, and the inference data, inference feature, variable data, and classifier of the present disclosure should not be construed as being limited due to the above-described examples.

Further, the machine learning model of the present disclosure may include classifiers whose number corresponds to the number of predetermined combinations of inference features. Specifically, the classifiers whose number corresponds to the number of predetermined combinations of the present disclosure combines the variable data of each of the inference features of the predetermined number of combinations to predict pass or fail for the noise level and output the prediction result.

For example, when the number of predetermined combinations is 2 and the number of inference features included in the first inference data is 8, the classifier may be constituted by 4 classifiers. Here, each of the four classifiers may output a prediction result predicting pass or fail based on two of the variable data of the eight inference features. Further, inference features and variable data input to each classifier may overlap. Specifically, first to second variable data are input to the first classifier to output a first prediction result, and second to fourth variable data may be input to the second classifier to output a second prediction result. Like the second variable data described above, variable data may be redundantly input into the first classifier and the second classifier. Further, one or more variable data that are input to each classifier may not overlap.

The above-described number of combinations, number of classifiers, number of inference features, and variable data are only examples, and the predetermined number of combinations, number of classifiers, inference features, and variable data of the present disclosure should not be construed as being limited due to the above-described examples.

In addition, those skilled in the art will be able to understand that explanations and examples of inputting the inference features of the above-described inference data into the machine learning model and outputting the prediction result may be described by explanations and examples of inputting the light features of the raw dataset into the machine learning model and outputting the prediction result.

According to an embodiment of the present disclosure, the machine learning model ensembles one or more prediction results for noise evaluation that are output through classifiers to finally generate output data representing the prediction result for noise evaluation for one detection device. That is, as described above, the machine learning model of the present disclosure ensembles prediction results obtained through one or more classifiers to predict whether one nucleic acid detection device will pass the noise level and represent the prediction as the output data.

The machine learning model is a supervised learning model using a plurality of learning data, and each of the plurality of learning data may include optic data obtained from a selected detection device as learning input data, and pass or fail indicating the noise level of the selected detection device as learning answer data.

The noise level of the selected detection device may be characterized by being obtained in relation to a negative control (NC) reaction and/or a positive control (PC) reaction executed by the selected detection device.

According to an embodiment of the present disclosure, when the machine learning model predicts that the nucleic acid detection device will not reach and pass the noise level, the machine learning model may generate output data predicting that a test for noise will be performed (740). Alternatively, when the machine learning model predicts that the nucleic acid detection device will reach and pass the noise level, the machine learning model may generate output data predicting that the test for noise will be omitted (750).

Accordingly, according to an embodiment of the present disclosure, the output data output by the machine learning model may include a result of predicting whether the nucleic acid detection device will pass the level for noise following the optic calibration. In addition, the output data of the present disclosure may include a result of predicting whether to omit the test for noise following the optic calibration for the nucleic acid detection device.

According to an additional embodiment of the present disclosure, the computing device may express step 730, indicated in FIG. 8 as passing or failing the noise level prediction, as three or more results. For example, the computing device may set two or more reference values for the noise level and output three or more results within a range determined by the two or more reference values. As an example, the three or more results may include conducting the test for noise, omitting the test for noise, and conducting a portion of the test for noise.

Further, according to an embodiment of the present disclosure, the computing device may generate learning data for learning the machine learning model of the present disclosure based on the output data obtained as described above. Specifically, the machine learning model of the present disclosure may be a supervised-learned model using variable data labeled with the pass or fail for the level of noise as the learning dataset. Here, the learning dataset may include variable data labeled with the result of evaluation of noise in relation to the output data, as described above. Further, the machine learning model of the present disclosure may be trained by inputting learning data consisting of variable data previously labeled by the user for the level of noise.

Further, the inference data or raw data of the present disclosure may include a plurality of variable data, and the machine learning model may include a neural network model. The neural network model of the present disclosure may include an input layer, at least one hidden layer, and an output layer. Here, the input layer may include input neurons whose number corresponds to the number of each of a plurality of variable data or a predetermined number of combinations of the plurality of variable data. Additionally, the output layer may include a plurality of output neurons, and each of the output neurons may represent pass or fail classes assigned to each variable data or each combination of variable data. Further, the output neurons included in the output layer may represent three or more classes including, for example, pass, partial pass, or fail.

As described above, according to some embodiments of the present disclosure, the test for noise may be omitted when the noise level is predicted to pass based on the result data of optic calibration performed before inferring the noise level through the machine learning model. As a result, time and human resources spent on noise evaluation may be saved, and the nucleic acid detection device may be quickly shipped.

FIG. 9 illustrates a flowchart of a method for inferring a noise level of a nucleic acid detection device according to an embodiment of the present disclosure.

According to an embodiment of the present disclosure, the computing device 100 may perform a method for inferring the noise level of the nucleic acid detection device. Specifically, the method of the present disclosure may include a step 810 of obtaining optic data containing information about light among raw data used for optic calibration of the nucleic acid detection device.

In the step of obtaining the optic data, optic data of an RFU value measured by irradiating light of a predetermined wavelength for a predetermined cycle to one component of the nucleic acid detection device may be obtained. Optic data including one or more optic data obtained as such may include one or more raw data obtained during a calibration process of irradiating light of a predetermined wavelength for a predetermined cycle.

Specifically, light may be irradiated to a reaction vessel containing a liquid medium and a dye, and the calibration process may include a step of adjusting the intensity of light irradiated to a sample in the nucleic acid detection device. Specifically, the light may be irradiated to the reaction vessel containing the liquid medium and the dye, and the optic calibration may include a step of adjusting the intensity of the light irradiated to a sample in the nucleic acid detection device.

The liquid medium is a medium that dissolves the dye. Typically, the liquid medium includes water and a buffer solution (TE buffer or PBS buffer).

Here, the sample may be a component of the nucleic acid detection device or a substance to be tested included in the component. Additionally, the optic calibration may include a step of correcting a result value of irradiated light.

Further, the method of the present disclosure may include a step 820 extracting a feature of noise from one or more parameters included in the optic data.

The light features may include at least one feature of: a first light feature representing the variance or standard deviation of the RFU values measured for one well, which is the component of the nucleic acid detection device; a second light feature representing an average of the RFU values for the component of the nucleic acid detection device; a third light feature representing a trend feature calculated by time series decomposition of the optic data; a fourth light feature representing a seasonality feature calculated by time series decomposition of the optic data; a fifth light feature representing remainder features other than the trend features and seasonality features calculated by time series decomposition of the optic data; or a sixth light feature including the variance of the residuals calculated by applying a first-order simple linear regression line to the optic data.

The method of the present disclosure may include a step 830 of generating inference data to be used to predict the noise level, based on at least one light feature. Here, the optic calibration may include a test that compares the RFU value measured in the nucleic acid detection device with the RFU value measured in the standard nucleic acid detection device that is different from the nucleic acid detection device. Further, prediction of the noise level may be performed with Negative Control (NC) or Positive Control (PC) contained in the sample reaction vessel of the nucleic acid detection device.

The step of generating the inference data may include a step of extracting, from the raw data, a light measurement value and a light feature of which relevancy with the performance of the nucleic acid detection device is equal to or larger than a threshold in noise generated by the nucleic acid detection device based on a predetermined p-value, a step of determining, as an inference feature, the light measurement value and the light feature of which relevancy with the performance of the nucleic acid detection device is equal to or larger than the threshold, and generating inference data including one or more inference features, and a step of generating a result of evaluation for noise based on variable data of the inference feature.

Here, the inference data may include inference features, and the machine learning model may include classifiers whose number corresponds to the number of predetermined combinations of inference features.

Additionally, the step 830 of generating the inference data described above may be omitted in the method of the present disclosure.

The method of the present disclosure may include a step 840 of inputting at least one of inference data or raw data into the machine learning model to obtain output data predicting the level of noise.

Predicting the level of noise may include a noise test associated with at least one of the Negative Control (NC) reaction or the Positive Control (PC) reaction for the nucleic acid detection device. The method of the present disclosure may be implemented through a machine learning model that predicts whether the noise level will pass by using the inference data or raw data as an input. The machine learning model is a pre-trained model that may generate an output about whether the noise level is passed based on the variable data of the light feature.

The output data may include a result of predicting whether to pass the level of noise following the optic calibration by the nucleic acid detection device. The output data may include a result of predicting whether to omit the test for noise following the optic calibration for the nucleic acid detection device.

The machine learning model may refer to any type of model capable of classifying input data through machine learning, and for example, the machine learning model may be a model that outputs output data using the SVM or partial least squares.

The inference data may include inference features and a plurality of variable data corresponding to the inference features, respectively, and the raw data may include light features and a plurality of variable data corresponding to the light features, respectively. The machine learning model may include a plurality of classifiers corresponding to at least one of the light measurement values and a plurality of light features and/or inference features, and each of the plurality of classifiers may output a result of passing or failing corresponding light measurement values, and light features and/or inference features, and the machine learning model ensembles outputs for the plurality of classifiers, respectively to generate output data predicting the level of noise. Here, one classifier may correspond to one light measurement value and one light feature.

The machine learning model may include classifiers whose number corresponds to a predetermined number of combinations of at least one of the inference features or the light features. Here, a machine learning-based classifier for classifying a combination for a plurality of variable data corresponding to one of the inference features or the light features may be used, and the number of classifiers used may be variable according to an aspect of implementation.

Further, the machine learning model may include classifiers whose number corresponds to a predetermined number of combinations of plurality of variable data corresponding to one of the inference features or the light features. Even here, a machine learning-based classifier for classifying a combination of a plurality of variable data may be used, and the number of used classifiers may be variable according to the aspect of the implementation.

The machine learning model may be a supervised-learned model using variable data labeled with the pass or fail for the level of noise as the learning dataset. In the learning step of the machine learning model, variable data labeled with a plurality of classes may be used. The plurality of classes may include, for example, a class corresponding to pass, partial pass, partial fail, or fail.

The machine learning model may include a neural network model, and the neural network model may include an input layer including input neurons of a number corresponding to the number of each of a plurality of variable data included in the inference data or the number of predetermined combinations of the plurality of variable data; at least one hidden layer; and an output layer including a plurality of output neurons. Here, each of the plurality of output neurons may represent pass or fail classes assigned to each variable data or each variable data combination. The neural network model is a model pre-trained to generate an output for a predefined class by using a plurality of variable data as an input.

In the method according to an embodiment of the present disclosure, since a noise test for the nucleic acid detection device predicted to have an excellent performance may be omitted, a test time required for each PCR device may be reduced by approximately 150 to 600 minutes. Therefore, the method according to an embodiment of the present disclosure may reduce the time required for the noise test and the manpower consumed, not only optimizing the inspection of the nucleic acid detection device, but also maximizing work efficiency of practitioners related to the nucleic acid detection device.

The steps of FIG. 9 described above may be changed in order as necessary, and at least one or more steps may be omitted or added. That is, the above-described steps are just an embodiment of the present disclosure, and the scope of the present disclosure is not limited thereto.

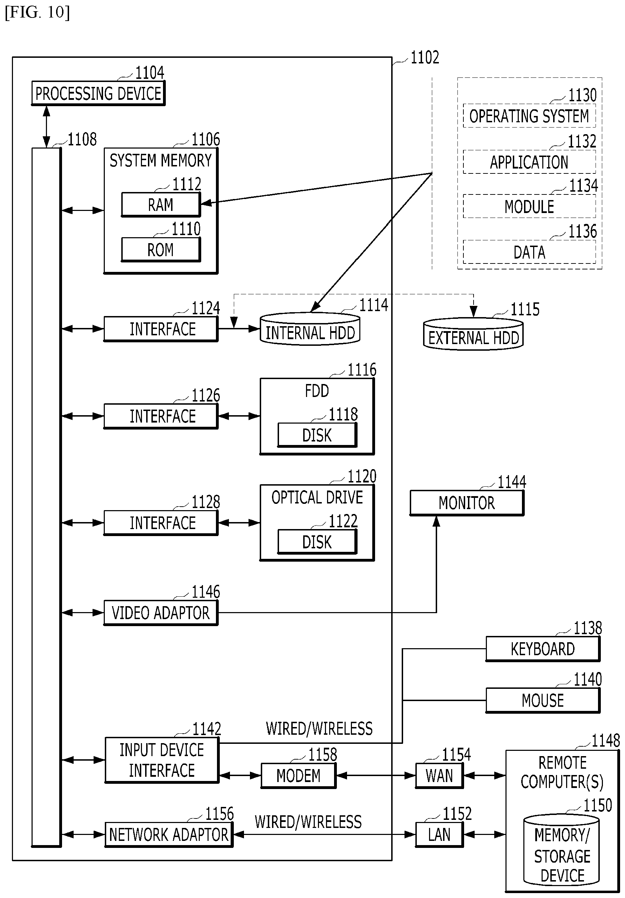

FIG. 10 is a simple and normal schematic view of an exemplary computing environment in which the embodiments of the present disclosure may be implemented.

It is described above that the present disclosure may be generally implemented by the computing device, but those skilled in the art will well know that the present disclosure may be implemented in association with a computer executable instruction and/or other program modules which may be executed on one or more computers and/or as a combination of hardware and software.

In general, the program module includes a routine, a program, a component, a data structure, and the like that execute a specific task or implement a specific abstract data type. Further, it will be well appreciated by those skilled in the art that the method of the present disclosure can be implemented by other computer system configurations including a personal computer, a handheld computing device, microprocessor-based or programmable home appliances, and others (the respective devices may operate in connection with one or more associated devices as well as a single-processor or multi-processor computer system, a mini computer, and a main frame computer.

The exemplary embodiments described in the present disclosure may also be implemented in a distributed computing environment in which predetermined tasks are performed by remote processing devices connected through a communication network. In the distributed computing environment, the program module may be positioned in both local and remote memory storage devices.

The computer generally includes various computer readable media. The computer includes, as a computer accessible medium, volatile and non-volatile media, transitory and non-transitory media, and mobile and non-mobile media. As a non-limiting example, the computer readable media may include both computer readable storage media and computer readable transmission media.

The computer readable storage media include volatile and non-volatile media, transitory and non-transitory media, and mobile and non-mobile media implemented by a predetermined method or technology for storing information such as a computer readable instruction, a data structure, a program module, or other data. The computer readable storage media include a RAM, a ROM, an EEPROM, a flash memory or other memory technologies, a CD-ROM, a digital video disk (DVD) or other optical disk storage devices, a magnetic cassette, a magnetic tape, a magnetic disk storage device or other magnetic storage devices or predetermined other media which may be accessed by the computer or may be used to store desired information, but are not limited thereto.

The computer readable transmission media generally implement the computer readable instruction, the data structure, the program module, or other data in a carrier wave or a modulated data signal such as other transport mechanism and include all information transfer media. The term "modulated data signal" means a signal acquired by setting or changing at least one of characteristics of the signal so as to encode information in the signal. As a non-limiting example, the computer readable transmission media include wired media such as a wired network or a direct-wired connection and wireless media such as acoustic, RF, infrared and other wireless media. A combination of any media among the aforementioned media is also included in the computer readable transmission media.

An exemplary environment 1100 that implements various aspects of the present disclosure including a computer 1102 is shown and the computer 1102 includes a processing device 1104, a system memory 1106, and a system bus 1108. The system bus 1108 connects system components including the system memory 1106 (not limited thereto) to the processing device 1104. The processing device 1104 may be a predetermined processor among various commercial processors. A dual processor and other multi-processor architectures may also be used as the processing device 1104.

The system bus 1108 may be any one of several types of bus structures which may be additionally interconnected to a local bus using any one of a memory bus, a peripheral device bus, and various commercial bus architectures. The system memory 1106 includes a read only memory (ROM) 1110 and a random access memory (RAM) 1112. A basic input/output system (BIOS) is stored in the non-volatile memories 1110 including the ROM, the EPROM, the EEPROM, and the like and the BIOS includes a basic routine that assists in transmitting information among components in the computer 1102 at a time such as in-starting. The RAM 1112 may also include a high-speed RAM including a static RAM for caching data, and the like.

The computer 1102 also includes an interior hard disk drive (HDD) 1114 (for example, EIDE and SATA), in which the interior hard disk drive 1114 may also be configured for an exterior purpose in an appropriate chassis (not illustrated), a magnetic floppy disk drive (FDD) 1116 (for example, for reading from or writing in a mobile diskette 1118), and an optical disk drive 1120 (for example, for reading a CD-ROM disk 1122 or reading from or writing in other high-capacity optical media such as the DVD, and the like). The hard disk drive 1114, the magnetic disk drive 1116, and the optical disk drive 1120 may be connected to the system bus 1108 by a hard disk drive interface 1124, a magnetic disk drive interface 1126, and an optical disk drive interface 1128, respectively. An interface 1124 for implementing an exterior drive includes at least one of a universal serial bus (USB) and an IEEE 1394 interface technology or both of them.

The drives and the computer readable media associated therewith provide non-volatile storage of the data, the data structure, the computer executable instruction, and others. In the case of the computer 1102, the drives and the media correspond to storing of predetermined data in an appropriate digital format. In the description of the computer readable media, the mobile optical media such as the HDD, the mobile magnetic disk, and the CD or the DVD are mentioned, but it will be well appreciated by those skilled in the art that other types of media readable by the computer such as a zip drive, a magnetic cassette, a flash memory card, a cartridge, and others may also be used in an exemplary operating environment and further, the predetermined media may include computer executable commands for executing the methods of the present disclosure.

Multiple program modules including an operating system 1130, one or more application programs 1132, other program module 1134, and program data 1136 may be stored in the drive and the RAM 1112. All or some of the operating system, the application, the module, and/or the data may also be cached in the RAM 1112. It will be well appreciated that the present disclosure may be implemented in operating systems which are commercially usable or a combination of the operating systems.

A user may input instructions and information in the computer 1102 through one or more wired/wireless input devices, for example, pointing devices such as a keyboard 1138 and a mouse 1140. Other input devices (not illustrated) may include a microphone, an IR remote controller, a joystick, a game pad, a stylus pen, a touch screen, and others. These and other input devices are often connected to the processing device 1104 through an input device interface 1142 connected to the system bus 1108, but may be connected by other interfaces including a parallel port, an IEEE 1394 serial port, a game port, a USB port, an IR interface, and others.

A monitor 1144 or other types of display devices are also connected to the system bus 1108 through interfaces such as a video adapter 1146, and the like. In addition to the monitor 1144, the computer generally includes other peripheral output devices (not illustrated) such as a speaker, a printer, others.

The computer 1102 may operate in a networked environment by using a logical connection to one or more remote computers including remote computer(s) 1148 through wired and/or wireless communication. The remote computer(s) 1148 may be a workstation, a computing device computer, a router, a personal computer, a portable computer, a micro-processor based entertainment apparatus, a peer device, or other general network nodes and generally includes multiple components or all of the components described with respect to the computer 1102, but only a memory storage device 1150 is illustrated for brief description. The illustrated logical connection includes a wired/wireless connection to a local area network (LAN) 1152 and/or a larger network, for example, a wide area network (WAN) 1154. The LAN and WAN networking environments are general environments in offices and companies and facilitate an enterprise-wide computer network such as Intranet, and all of them may be connected to a worldwide computer network, for example, the Internet.

When the computer 1102 is used in the LAN networking environment, the computer 1102 is connected to a local network 1152 through a wired and/or wireless communication network interface or an adapter 1156. The adapter 1156 may facilitate the wired or wireless communication to the LAN 1152 and the LAN 1152 also includes a wireless access point installed therein in order to communicate with the wireless adapter 1156. When the computer 1102 is used in the WAN networking environment, the computer 1102 may include a modem 1158 or has other means that configure communication through the WAN 1154 such as connection to a communication computing device on the WAN 1154 or connection through the Internet. The modem 1158 which may be an internal or external and wired or wireless device is connected to the system bus 1108 through the serial port interface 1142. In the networked environment, the program modules described with respect to the computer 1102 or some thereof may be stored in the remote memory/storage device 1150. It will be well known that an illustrated network connection is exemplary and other means configuring a communication link among computers may be used.

The computer 1102 performs an operation of communicating with predetermined wireless devices or entities which are disposed and operated by the wireless communication, for example, the printer, a scanner, a desktop and/or a portable computer, a portable data assistant (PDA), a communication satellite, predetermined equipment or place associated with a wireless detectable tag, and a telephone. This at least includes wireless fidelity (Wi-Fi) and Bluetooth wireless technology. Accordingly, communication may be a predefined structure like the network in the related art or just ad hoc communication between at least two devices.

The wireless fidelity (Wi-Fi) enables connection to the Internet, and the like without a wired cable. The Wi-Fi is a wireless technology such as the device, for example, a cellular phone which enables the computer to transmit and receive data indoors or outdoors, that is, anywhere in a communication range of a base station. The Wi-Fi network uses a wireless technology called IEEE 802.11(a, b, g, and others) in order to provide safe, reliable, and high-speed wireless connection. The Wi-Fi may be used to connect the computers to each other or the Internet and the wired network (using IEEE 802.3 or Ethernet). The Wi-Fi network may operate, for example,at a data rate of 11 Mbps (802.11a) or 54 Mbps (802.11b) in unlicensed 2.4 and 5GHz wireless bands or operate in a product including both bands (dual bands).

It will be appreciated by those skilled in the art that information and signals may be expressed by using various different predetermined technologies and techniques. For example, data, instructions, commands, information, signals, bits, symbols, and chips which may be referred in the above description may be expressed by voltages, currents, electromagnetic waves, magnetic fields or particles, optical fields or particles, or predetermined combinations thereof.

It may be appreciated by those skilled in the art that various exemplary logical blocks, modules, processors, means, circuits, and algorithm steps described in association with the exemplary embodiments disclosed herein may be implemented by electronic hardware, various types of programs or design codes (for easy description, herein, designated as software), or a combination of all of them. In order to clearly describe the intercompatibility of the hardware and the software, various exemplary components, blocks, modules, circuits, and steps have been generally described above in association with functions thereof. Whether the functions are implemented as the hardware or software depends on design restrictions given to a specific application and an entire system. Those skilled in the art of the present disclosure may implement functions described by various methods with respect to each specific application, but it should not be interpreted that the implementation determination departs from the scope of the present disclosure.

Various embodiments presented herein may be implemented as manufactured articles using a method, a device, or a standard programming and/or engineering technique. The term manufactured article includes a computer program, a carrier, or a medium which is accessible by a predetermined computer-readable storage device. For example, a computer-readable storage medium includes a magnetic storage device (for example, a hard disk, a floppy disk, a magnetic strip, or the like), an optical disk (for example, a CD, a DVD, or the like), a smart card, and a flash memory device (for example, an EEPROM, a card, a stick, a key drive, or the like), but is not limited thereto. Further, various storage media presented herein include one or more devices and/or other machine-readable media for storing information.

It will be appreciated that a specific order or a hierarchical structure of steps in the presented processes is one example of exemplary accesses. It will be appreciated that the specific order or the hierarchical structure of the steps in the processes within the scope of the present disclosure may be rearranged based on design priorities. Appended method claims provide elements of various steps in a sample order, but the method claims are not limited to the presented specific order or hierarchical structure. The description of the presented embodiments is provided so that those skilled in the art of the present disclosure use or implement the present disclosure. Various modifications of the exemplary embodiments will be apparent to those skilled in the art and general principles defined herein can be applied to other exemplary embodiments without departing from the scope of the present disclosure. Therefore, the present disclosure is not limited to the embodiments presented herein, but should be interpreted within the widest range which is coherent with the principles and new features presented herein.

## Claims

1. A method for inferring a noise level of a detection device for detecting a presence or absence of a target analyte in a sample based on a signal generated dependent on the presence or absence of the target analyte, the method for inferring a noise level comprising:
obtaining optic data with information about light among raw data used for an optic calibration of the detection device; and
inferring a level of noise generated in the detection device by using a pre-trained machine learning model with the optic data as input data.

2. The method for inferring a noise level of claim 1, wherein the optic data comprises light measurement values obtained by irradiating light with a pre-determined wavelength over pre-determined cycles and light features obtained by mathematically processing the light measurement values.

3. The method for inferring a noise level of claim 2, wherein the irradiation of light is done to a reaction vessel comprising a liquid medium and a dye.

4. The method for inferring a noise level of claim 1, wherein the optic calibration is adjusting an intensity of the irradiated light based on a comparison result between the optic data and a predetermined reference value.

5. The method for inferring a noise level of claim 1, wherein the optic calibration is adjusting a light quantity measurement sensitivity of the detection device based on a comparison result between the optic data and a predetermined reference value.

6. The method for inferring a noise level of claim 1, wherein the method further comprises:
extracting light measurement values and light features obtained by mathematically processing the light measurement values from the optic data; and
inputting the light measurement values and the light features to the machine learning model for inferring the noise level.

7. The method for inferring a noise level of claim 6, wherein the light features comprise at least one selected from the group consisting of:
a first light feature comprising a variance or standard deviation for the optic data;
a second light feature comprising an average of the optic data;
a third light feature comprising a trend calculated by time-series decomposition of the optic data;
a fourth light feature comprising seasonality calculated by time-series decomposition of the optic data;
a fifth light feature comprising a remainder obtained by subtracting the third and fourth light features from the optic data;
a sixth light feature comprising a variance of a residual calculated by applying a linear regression line to the optic data; and
a combination thereof.

8. The method for inferring a noise level of claim 6, wherein the extraction produces a plurality of light features of different types,
wherein the machine learning model comprises at least three classifiers corresponding to the light measurement values and the plurality of light features; wherein one classifier of the at least three classifiers corresponds to one light measurement value and one or at least two light features,
wherein each of the at least three classifiers, once inputted with its corresponding light measurement values and light features, outputs either PASS or FAIL as a provisional noise level of the detection device,
wherein the machine learning model infers the noise level of the detection device by ensembling outputs of each of the at least three classifiers.

9. The method for inferring a noise level of claim 6, wherein the extraction produces a plurality of light features of different types,
wherein the inference of the noise level further comprises extracting at least one inference feature from the light measurement values and a plurality of light features based on a predetermined p-value,
wherein the machine learning model is inputted with at least one inference feature to infer the noise level.

10. The method for inferring a noise level of claim 1, wherein the machine learning model comprises a model inferring the noise level by using SVM(Support vector Machine) or Partial Least Squares.

11. The method for inferring a noise level of claim 1, wherein the machine learning model is a supervised-learned model with a plurality of learning data,
wherein each of the plurality of learning data comprises:
optic data obtained from selected detection devices as learning input data and;
PASS or FAIL indicating the noise level of the selected detection
devices as learning answer data.

12. The method for inferring a noise level of claim 11, wherein the noise level of the selected detection devices is obtained with regard to negative control reaction and/or positive control reaction run on the selected detection devices.

13. The method for inferring a noise level of claim 1, wherein the optic data comprises light measurement values and a plurality of light features of different types,
wherein the machine learning model comprises a neural network model comprising an input layer, at least one hidden layer and an output layer,
wherein the input layer comprises input nodes corresponding to light measurement values and at least one of one or more light features, and
wherein the output layer comprises output nodes corresponding to PASS and FAIL.

14. A computer readable medium storing a computer program,
wherein the computer program comprises commands for allowing, when executed by one or more processors, the one or more processors to perform a method for inferring a result of evaluation for noise of a detection device for detecting a presence or absence of a target analyte in a sample based on a signal generated dependent on presence or absence of the target analyte,
wherein the method comprises:
obtaining optic data used for a calibration process of the detection device; and
inferring a noise level generated in the detection device by using a pre-trained machine learning model with the optic data as input data.

15. a computing device for performing a method for inferring a noise level generated in a detection device for detecting a presence or absence of a target analyte in a sample based on a signal generated dependent on the presence or absence of the target analyte,
wherein the computing device comprises:
an input unit obtaining optic data used for an optic calibration of the detection device; and
a noise level inference unit inferring the noise level generated in the detection device by using a pre-trained machine learning model with the optic data as input data.
